# EUROPEAN PATENT APPLICATION

(11) **EP 4 722 192 A1**
(43) Date of publication of application: **08.04.2026**
(21) Application number: 24204528.4
(22) Date of filing: 03.10.2024
(51) Int. Cl.: C07C 231/10, C07C 231/18, C07C 233/65, C07C 209/62, C07C 211/42, C07D 251/18

(54) **PROCESS FOR THE PREPARATION OF AMIDE INTERMEDIATES**

(71) Applicant: Adama Agan Ltd., 7710201 Ashdod (IL)
(72) Inventor: BAR NAHUM, Itsik, 7691000 Kfar-Gibton (IL); PARNES, Regev, 8531500 Kibbutz Mishmar Hanegev (IL)
(74) Representative: Modiano, Gabriella Diana

(57) **Abstract**

The present disclosure relates a process for the preparation of amides as intermediates for the synthesis of triazines, which are suitable for use as herbicides for controlling weeds.

## Description

### TECHNICAL FIELD

The present disclosure relates to the field of agrochemistry. More specifically, it is directed to a process for preparation of amides as intermediates for the synthesis of triazines, which are suitable as herbicides for controlling weeds.

### BACKGROUND

Triazine compounds are a class of compounds suitable for being used as herbicides. Triazine compounds such as Atrazine, Ametryne, Indaziflam or Triaziflam are among the compounds that are used as herbicides.

The synthesis of Indaziflam was disclosed for the first time in document WO04/069814 A1. A compound of Formula (V) is mentioned as starting material in one of the alternative syntheses described in said prior art document. 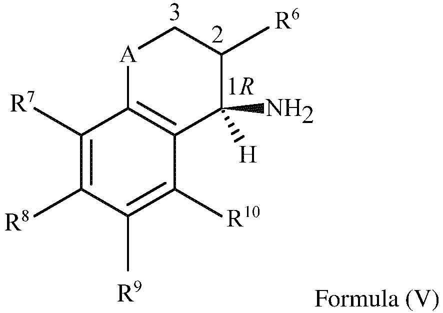 wherein:
R⁶ is (C₁-C₆)alkyl;
R⁷, R⁸, R⁹ and R¹⁰ are each independently H, (C₁-C₄)alkyl, (C₁-C₃)haloalkyl, halogen, (C₁-C₃)alkoxy, (C₁-C₃)haloalkoxy or CN;
A is CH₂, O or a direct bond, according to document WO 04/069814 A1.

Compounds of Formula (V) were described to be prepared according to known methods, for example by reductive amination of ketones or the corresponding oximes. However, this synthesis requires the separation of mixtures of 4 isomers, i.e., cis/trans isomers and enantiomers, as disclosed in the examples of that document.

The synthesis of a compound of Formula (1R,2S)-2,6-dimethyl-2,3-dihydro-1-indan-1-amine was also described in the document WO 2015/113903 A1: 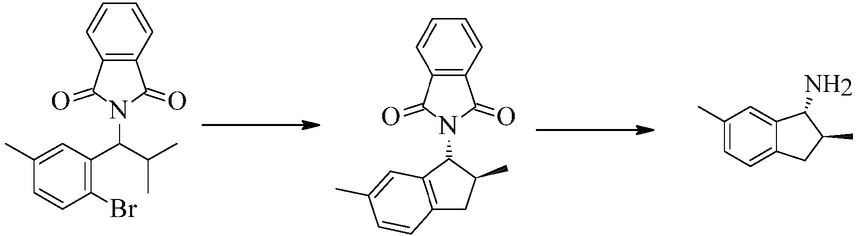 (1*R*,2*S*)-2,6-dimethyl-2,3-dihydro-1-indan-1-amine was prepared from 2-[1-(2-bromo-5-methylphenyl)-2-methylpropyl]-1H-isoindole-1,3(2H)-dione via 2-[(1*R*,2*S*)-2,6-dimethyl-2,3-dihydro-1H-inden-1-yl]-1H-isoindole-1,3(2H)-dione by using a palladium tetraphenyl-phosphine catalyst with a yield of 55% from the halogenated compound.

An additional approach described in document CN 108794339, which refers to the separation of (1R,2S)-2,6-dimethyl-1-aminoindan (I) from the mixture (III) of four isomers as raw material. Initially, an enantiomer mixture of compounds of Formula (II), enriched in the trans-isomers, is prepared through a method of hydrogenation at the presence of a palladium catalyst, or a method of salifying crystallization with achiral organic acid or the two methods; using R-mandelic acid to split an enantiomer mixture of Formula (II) to obtain enantiomer RS-(I) enriched (1*R*,2*S*)-2,6-dimethyl-1-aminoindan. Waste isomer generated in the preparation method can be converted into the compound shown as the Formula (II) and enriched with the trans-isomer through a palladium catalytic hydrogenation method for recycling.

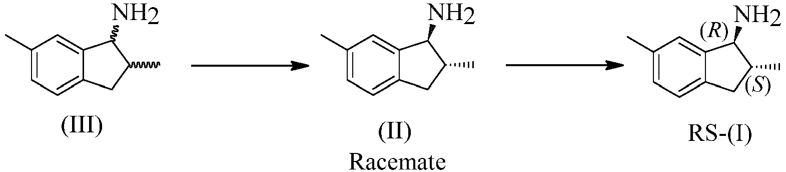

Bayer's process for the synthesis of Indaziflam is described in document US 2004/157739. According to this process, in Step 1, a homogeneous mixture of (*1*R,2*S*)-1-amino-2-methylindane hydrochloride and 1-cyanoguanidine in 1,3-dichlorobenzene was heated at 140-150° C. The cooled mixture was diluted with toluene and the solid filtered off to give solid (1*R*,2*S*)-1-(bisguanidino)-2-methylindane monohydrochloride.

In Step 2, a 30% solution of sodium methoxide in methanol was added to a stirred suspension of (1*R*,2*S*)-1-(bisguanidino)-2-methylindane monohydrochloride in methanol. Then methyl-(2*R*)-2-fluoropropanoate was added at room temperature, followed by an additional quantity of 30% solution of sodium methoxide. The residue was purified by column chromatography, eluting with a mixture of ethyl acetate:heptane (7:3) as eluent to give 2-amino-4-[(1*R*,2*S*)-2-methyl-1-indanylamino]-6-[(1*R*)-1-fluoroethyl]-1,3,5-triazine (Indaziflam).

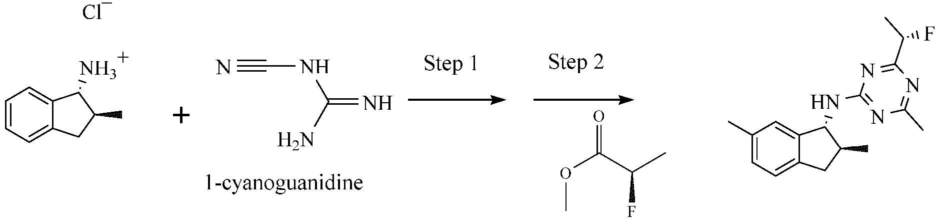

Therefore, due to the complex synthesis of Indaziflam intermediate, (1R,2S)-2,6-dimethyl-2,3-dihydro-1H-inden-1-amine, there is a need in the art for a simple to operate reaction, with high enantioselectivity and/or yield, and from a simple and available starting material.

### SUMMARY

A first aspect of the invention relates to a process for the preparation of an amide compound of Formula (I): 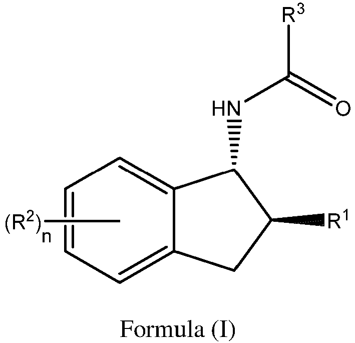 wherein:
R¹ and R² may each independently be hydrogen or an optionally substituted C₁-C₄ alkyl group, and wherein R¹ and R² are not both hydrogen;
R³ may be an optionally substituted C₁-C₆ alkyl group, an optionally substituted C₁-C₆ alkenyl group, an optionally substituted (C₁-C₂-alkyl)-O-(C₁-C₄-alkyl) group, an optionally substituted aryl group, a phenyl or an aralkyl group with a C₁-C₄ alkyl chain;
wherein the optional substituent may be selected from the group comprising halogen, aliphatic, halo aliphatic, alicyclic, alkoxy, thioalkyl, hydroxy, cyano, aryl and nitro group;
n may be 1, 2 or 3;
which comprises the amidation of an indene compound of Formula (II) 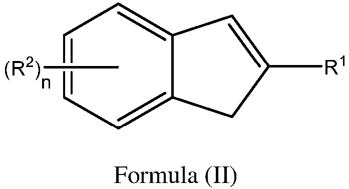
with an amidation agent selected from the compound of Formula (III), 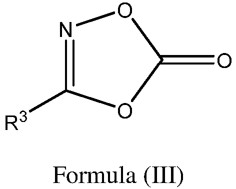
wherein the amidation may be in the presence of a hydrosilane reducing agent and a catalyst, wherein the catalyst may be a complex of a transition metal with a monodentate, bidentate or polydentate chiral ligand.

A second aspect of the invention relates to a compound of Formula (IV), 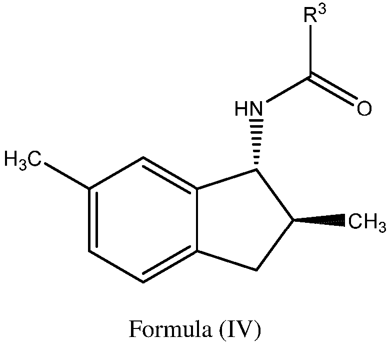 wherein R³ may be an optionally substituted C₁-C₆ alkyl group, an optionally substituted C₁-C₆ alkenyl group, an optionally substituted (C₁-C₂-alkyl)-O-(C₁-C₄-alkyl) group, an aryl group selected from the group consisting of tolyl, naphthyl or mesityl, or an aralkyl group with a C₁-C₄ alkyl chain;
wherein the optional substituent may be selected from the group comprising halogen, aliphatic, halo aliphatic, alicyclic, alkoxy, thioalkyl, hydroxy, cyano, aryl and nitro group.

A third aspect of the invention relates to a process for the preparation of an amide compound of Formula (I): 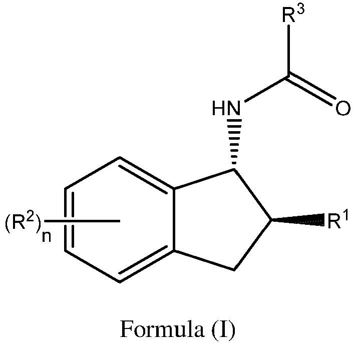 which comprises:
a) a first step of dehydration of an indanol compound of Formula (V) 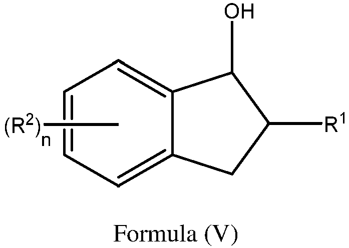 to prepare a compound of Formula (II) 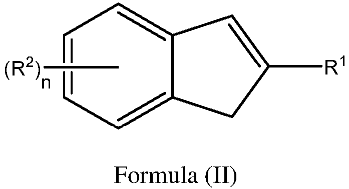
b) a second step of amidation of the indene compound of Formula (II);
   wherein:
   R¹ and R² may each independently be hydrogen or an optionally substituted C₁-C₄ alkyl group, and wherein R¹ and R² are not both hydrogen;
   R³ may be an optionally substituted C₁-C₆ alkyl group, an optionally substituted C₁-C₆ alkenyl group, an optionally substituted (C₁-C₂-alkyl)-O-(C₁-C₄-alkyl) group, an optionally substituted aryl group, a phenyl or an aralkyl group with a C₁-C₄ alkyl chain;
   wherein the optional substituent is selected from the group comprising halogen, aliphatic, halo aliphatic, alicyclic, alkoxy, thioalkyl, hydroxy, cyano, aryl and nitro group;
   n may be 1, 2 or 3.

A fourth aspect of the invention is the preparation of the compound of Formula (VII), (1R,2S)-1-amido-2,6-dimethylindane, by hydrolysis of a compound of Formula (IV) in water with a hydrolyzing agent which is a base or an acid, 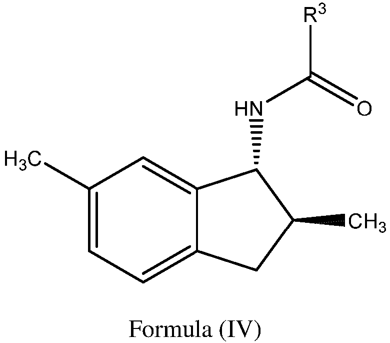 wherein the substituent R³ in compound of Formula (IV) may be an optionally substituted C₁-C₆ alkyl group, an optionally substituted C₁-C₆ alkenyl group, an optionally substituted (C₁-C₂-alkyl)-O-(C₁-C₄-alkyl) group, an optionally substituted aryl group, a phenyl or an aralkyl group with a C₁-C₄ alkyl chain;
wherein the optional substituent may be selected from the group comprising halogen, aliphatic, halo aliphatic, alicyclic, alkoxy, thioalkyl, hydroxy, cyano, aryl and nitro group.

A fifth aspect of the invention is a process for the preparation of Indaziflam, the process comprising preparing a compound of Formula (IV).

A sixth aspect of the invention is a process for preparation of Indaziflam comprising:
a) preparing compound of Formula (IV);
b) providing reaction conditions for preparation of Indaziflam.

A seventh aspect of the invention is a process for the preparation of Indaziflam, the process comprising:
a. reacting a compound of Formula (IV) or the compound of Formula (VII), with 1-cyanoguanidine in the presence of at least one solvent to obtain (1*R*,2*S*)-1-(bisguanidino)-2,6-dimethylindane; and
b. reacting said (1*R*,2*S*)-1-(bisguanidino)-2,6-dimethylindane with methyl-(2*R*)-2-fluoropropanoate in the presence of a base and in the presence of a solvent,
wherein when the reacting compound is a compound of Formula (IV), the compound obtained from the reaction of the compound of Formula (IV) with 1-cyanoguanidine is hydrolyzed with water and a hydrolyzing agent to obtain (1*R*,2*S*)-1-(bisguanidino)-2,6-dimethylindane before reacting with methyl-(2*R*)-2-fluoropropanoate.

An eighth aspect of the invention is a process for the preparation of Indaziflam, the process comprising:
a. reacting a compound of Formula (IV) or the compound of Formula (VII), with 1-cyanoguanidine and a tri(alkyl) aluminum (III) salt in the presence of a protic solvent selected from the group consisting of alcohols or amines, to obtain a biguanidino-aluminum complex; and
b. reacting said biguanidino-aluminum complex with methyl-(2*R*)-2-fluoropropanoate,
wherein when the reacting compound is a compound of Formula (IV), the biguanidino-aluminum complex obtained from the reaction of the compound of Formula (IV) with 1-cyanoguanidine and the tri(alkyl) aluminum (III) salt is hydrolyzed with water and a hydrolyzing agent before reacting with methyl-(2*R*)-2-fluoropropanoate.

Additional features and advantages of the present disclosure will become apparent to those skilled in the art upon consideration of the following detailed description of the illustrative embodiments exemplifying the best mode of carrying out the utility described herein as presently perceived.

### DETAILED DESCRIPTION

### Definitions

Embodiments of the present disclosure are discussed in detail below. In describing the embodiments, specific terminology is employed for the sake of clarity. However, the disclosure is not intended to be limited to the specific terminology so selected. While specific exemplary embodiments are discussed, it should be understood that this is done for illustration purposes only. A person skilled in the relevant art will recognize that other components and configurations can be used without parting from the spirit and scope of the disclosure. While a number of embodiments and features are described herein, it is to be understood that the various features of the disclosure and aspects of embodiments, even if described separately, may be combined unless mutually exclusive or contrary to the specific description. All references cited herein are incorporated by reference as if each had been individually incorporated.

As used herein, the transitional term "comprising" or "that comprises", which is synonymous with "including," or "containing," is inclusive or open-ended and does not exclude additional, un-recited elements or method steps. However, in each recitation of "comprising" herein, it is intended that the term also encompass, as alternative embodiments, the phrases "consisting essentially of" and "consisting of", where "consisting of" excludes any element or step not specified and "consisting essentially of" permits the inclusion of additional un-recited elements or steps that do not materially affect the essential or basic and novel characteristics of the composition or method under consideration.

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Prior to setting forth the present subject matter in detail, the definitions of certain terms to be used herein are provided. Unless defined otherwise, all technical and scientific terms used herein have the same meaning as is commonly understood by one of skill in the art to which this subject matter pertains.

The term "a" or "an" as used herein may include the singular and the plural, unless specifically stated otherwise. Therefore, the terms "a", "an" or "at least one" can be used interchangeably in this application.

As used herein, the term "compound" may include all forms thereof including, but not limited to, salts, solid forms such as amorphous, crystalline, solvate or hydrate.

As used herein, the term "aryl group" may refer to an optionally substituted aromatic carbocyclic or heterocyclic group which has between 4 and 10, between 5 and 10, between 5 and 8 carbon atoms, which comprises 1 aromatic ring or 2 fused aromatic rings, wherein the optional substituents are selected from the group including halogen, aliphatic, halo aliphatic, alkoxy, thioalkyl, cyano or nitro groups. In the case of a heterocyclic aryl group, i.e., "heteroaryl group", 1, 2 or 3 of the atoms in the ring or rings of the aryl group, is an element other than carbon, such as nitrogen, oxygen or sulfur.

The term "aralkyl group" may refer to an aryl-substituted alkyl group.

The term "alkoxy group" may refer to an alkyl group bound to oxygen.

The term "aryloxy group" may refer to an aryl group bound to oxygen.

The term "aralkoxy group" as used herein in accordance with some embodiments, may refer to an aralkyl group bound to oxygen.

The term "thioalkyl group" as used herein in accordance with some embodiments, may refer to an alkyl group bound to sulfur. The term "halo aliphatic group" as used herein in accordance with some embodiments, may refer to an aliphatic group substituted with at least one halogen atom.

The term "aliphatic group" as used herein in accordance with some embodiments may cover, for example and not restricted to, the linear or branched alkyl, alkenyl and alkynyl groups.

The term "alkyl group" may refer to a saturated, linear or branched group, which has between 1 and 24, between 1 and 16, between 1 and 14, between 1 and 12, or 1, 2, 3, 4, 5 or 6 carbon atoms and is bound to the rest of the molecule by a single bond, including, for example and not restricted to, methyl, ethyl, isopropyl, isobutyl, tert-butyl, heptyl, octyl, decyl, dodecyl, hexadecyl, octadecyl, amyl, 2-ethylhexyl, 2-methylbutyl, 5-methylhexyl and similar.

The term "alkenyl group" as used herein in accordance with some embodiments, may refer to a linear or branched group, which has between 2 and 24, between 2 and 16, between 2 and 14, between 2 and 12, or 2, 3, 4, 5 or 6 carbon atoms, with 1, 2 or 3 carbon-carbon double bonds, conjugated or unconjugated, which is bound to the rest of the molecule by a single bond, including, for example and not restricted to, ethenyl, 1-propenyl, 2-propenyl, 1-butenyl, 2-butenyl, pentenyl, and similar groups.

The term "alkynyl group" as used herein in accordance with some embodiments, may refer to a linear or branched group, which has between 2 and 24, between 2 and 16, between 2 and 14, between 2 and 12, or 2, 3, 4, 5 or 6 carbon atoms, with 1, 2 or 3 carbon-carbon triple bonds, conjugated or unconjugated, which is bound to the rest of the molecule by a single bond, including, for example and not restricted to, an ethynyl, 1-propynyl, 2-propynyl, 1-butynyl, 2-butynyl, pentynyl, such as 1-pentynyl, and similar.

The term "alicyclic group" as used in accordance with some embodiments, may cover, for example and not restricted to, cycloalkyl, cycloalkenyl and/or cycloalkynyl groups.

The term "cycloalkyl" as used herein in accordance with some embodiments, may refer to a saturated mono- or polycyclic aliphatic group which has between 3 and 24, between 3 and 16, between 3 and 14, between 3 and 12, or 3, 4, 5 or 6 carbon atoms and which is bound to the rest of the molecule by a single bond, including, for example and not restricted to, cyclopropyl, cyclobutyl, cyclopentyl, cyclohexyl, cycloheptyl, methyl cyclohexyl, dimethyl cyclohexyl, octahydroindene, decahydronaphthalene, dodecahydrophenalene and/or similar.

The term "cycloalkenyl" as used herein in accordance with some embodiments, may refer to a non-aromatic mono- or polycyclic aliphatic group which has between 5 and 24, between 5 and 16, between 5 and 14, between 5 and 12, or 5 or 6 carbon atoms, with 1, 2 or 3 carbon-carbon double bonds, conjugated or unconjugated, and which is bound to the rest of the molecule by a single bond, including, for example and not restricted to, the cyclopent-1-en-1-yl group and/or similar.

The term "cycloalkynyl" as used herein in accordance with some embodiments, may refer to a non-aromatic mono- or polycyclic aliphatic group which has between 8 and 24, between 8 and 16, between 8 and 14, between 8 and 12, or 8 or 9 carbon atoms, with 1, 2 or 3 carbon-carbon triple bonds, conjugated or unconjugated, and which is bound to the rest of the molecule by a single bond, including, for example and not restricted to, the cyclooct-2-in-1-yl group and/or similar.

The term "hydrosilane" as used herein in accordance with some embodiments, may refer to any organic tetravalent silicon compound containing one or more Si-H bonds. The term "alkoxy hydrosilane" as used herein refers to any organic tetravalent silicon compound containing one or more Si-H bonds and one or more alkoxy groups bound to silicon. For example, and not restricted to, methyldimethoxysilane, trimethoxysilane, dimethoxysilane, diethyoxysilane, triethoxysilane, poly(methylhydrosilane), oligosilanes and/or similar.

The term "hydrosiloxane" as used herein in accordance with some embodiments, may refer to any organosilicon compound containing at least one Si-O-Si group where each Si atom carries two aliphatic or aralkyl groups. The term "alkoxy hydrosiloxanes" as used herein refers to any organosilicon compound containing at least one Si-O-Si group where each Si atom carries two aliphatic or aralkyl groups, at least one of which is an alkoxy group. For example, and not restricted to, polymethylhydrosiloxane (PMHS), phenylsiloxane, oligosiloxane, methyldimethoxysiloxane, trimethoxysiloxane, dimethoxysiloxane, diethyoxysiloxane, and/or similar.

The terms "monodentate chiral ligand", "bidentate chiral ligand" and "polydentate chiral ligand" as used herein in accordance with some embodiments, may refer to a chiral ligand bound to the rest of the molecule by a single bond or a double bond, and the rest of the molecule may be an organic or organometallic molecule, wherein the chiral ligand include a single donor atom, two donor atoms or multiple donor atoms.

Non-limiting examples of monodentate ligands are: [(1*R*)-2'-methoxy[1,1'-binaphthalen]-2-yl]diphenylphosphine, (1*R*)-[1,1'-binaphthalen]-2-yldiphenyl-phosphine, [(2*S*)-2-(1-naphthalenyl)[1,1'-biphenyl]-3-yl]diphenyl-phosphine, [(1*S*,2*S*,5*R*)-5-methyl-2-(1-methylethyl)cyclohexyl]diphenylphosphine, 3-(diphenylphosphino)-2-[(*R*)-methoxyphenylmethyl]-(3*S*)-ferrocene, derivatives or enantiomers thereof and/or similar.

The term "phosphorous-containing bidentate chiral ligand" as used herein in accordance with some embodiments, may refer to a bidentate chiral ligand containing one or more phosphorous atoms, which is bound to the rest of the molecule by a single bond or a double bond, and the rest of the molecule may be an organic or organometallic molecule. For example, and not restricted to: (+)-1,2-bis((2*S*,5*S*)-2,5-diphenylphospholano)ethane, (+)-1,2-bis((2*S*,5*S*)-2,5-dipropyl phospholano)ethane, (-)-1,2-bis[(2*R*,5*R*)-2,5-diethylphospholanol benzene, (-)-1,2-bis[(2*R*,5*R*)-2,5-dimethylphospholano]benzene, (-)-1,2-bis[(2*R*,5*R*)-2,5-dimethylphospholano]benzene, (-)-1,2-bis[(2*S*,5*S*)-2,5-dimethylphospholano]ethane, 1-(*S*)-1,2-bis(diphenylphosphino)ferrocenyl]ethylamine, 1,2-bis(diphenylphosphino) ethane (DPPE), 1,2-bis(dicyclohexylphosphino)ethane (DCPE), 1,3-bis(diphenyl phosphino)propane (DPPP), 1,4-bis(diphenylphosphino)butane (DPPB), 1,1'-bis(diphenylphosphino)ferrocene (DPPF), (+)-2,2-dimethyl-4,5-((diphenylphosphino) dimethyl)dioxolane, (2*S*,4*S*)-(-)-4-diphenylphosphino-2-(diphenylphosphinomethyl) pyrrolidine, 2-(bucyclohexane)-1-(diphenylphosphino)ferrocene, 2,2'-bis(diphenyl phosphino)-1,1'-binaphthyl) (BINAP), (-)-1,2-bis[(2*R*,5*R*)-2,5-dimethylphospholano] benzene, (2*R*,2'*R*,5*R*,5*'*R)-2,2',5,5'-tetramethyl-1,1'-(o-phenylene)diphospholane (Me-Duphos), Xyliphos, Josiphos, derivatives or enantiomers thereof and/or similar.

The term "nitrogen containing bi-dentate chiral ligand" as used herein in accordance with some embodiments, may refer to a bidentate chiral ligand containing one or more nitrogen atoms, which is bound to the rest of the molecule by a single bond or a double bond, and the rest of the molecule may be an organic or organometallic molecule.

The term "transition metal" as used herein in accordance with some embodiments may refer to a metal selected from the group consisting of transition metals on the Periodic table with an oxidation state of -4, -3, -2, -1, 0, +1, +2, +3, +4, +5, +6 or +7.

The term "cationic transition metal" as used herein in accordance with some embodiments may refer to a positively charged metal selected from the group consisting of transition metals on the Periodic table with an oxidation state of +1, +2, +3, +4, +5, +6 or +7. Non-limiting examples of cationic transition metals are: Cu⁺, Ni⁺, Fe⁺, Ru⁺, Cr⁺, Mo⁺, W⁺, Co⁺, Mn⁺, Cu²⁺, Ni²⁺, Pd²⁺, Pt²⁺, Fe²⁺, Ru²⁺, Cr²⁺, Mo²⁺, W²⁺, Co²⁺, Mn²⁺, Cu³⁺ , Ni³⁺, Pd³⁺, Fe³⁺, Ru³⁺, Cr³⁺ , Mo³⁺, W³⁺,Co³⁺, Mn³⁺, Ni⁴⁺, Pt⁴⁺, Fe⁴⁺, Ru⁴⁺, Cr⁴⁺, Mo⁴⁺, W⁴⁺, Mn⁴⁺ and/or similar.

All abbreviations, including chemical abbreviations, have their commonly accepted meanings.

For purposes of better understanding the present teachings and in no way limiting the scope of the teachings, unless otherwise indicated, all numbers expressing quantities, percentages or proportions, and other numerical values used in the specification and claims, are to be understood as being modified in all instances by the term "about". Accordingly, unless indicated to the contrary, the numerical parameters set forth in the following specification and attached claims are approximations that may vary depending upon the desired properties sought to be obtained. At the very least, each numerical parameter should at least be construed in light of the number of reported significant digits and by applying ordinary rounding techniques. In this regard, used of the term "about" herein specifically includes ±10% from the indicated values in the range. In addition, the endpoints of all ranges directed to the same component or property herein are inclusive of the endpoints, are independently combinable, and include all intermediate points and ranges. Similarly, the ranges and amounts for each element of the technology described herein can be used together with ranges or amounts for any of the other elements.

Any range or desired value given herein may be extended or altered without losing the effects sought, as is apparent to the skilled person for an understanding of the teachings herein.

The present disclosure provides an alternative to the existing methods for the preparation of Indaziflam intermediates. It is further an object of the present disclosure, to provide a process which solves at least some of the drawbacks of prior art methods such as non-enantioselective synthesis and/or low yield. Advantageously, a chiral synthesis with good enantiomeric excess and/or yield may be used to prepare Indaziflam intermediates.

### Process for the preparation of amide compounds of Formula (I)

A first aspect of the invention relates to a process for the preparation of an amide compound of Formula (I): 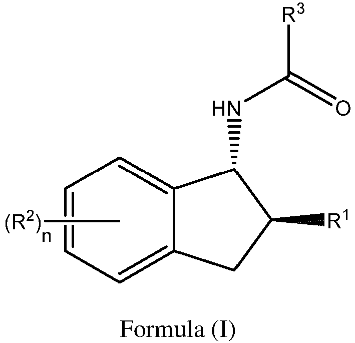 wherein:
R¹ and R² may each independently be hydrogen or an optionally substituted C₁-C₄ alkyl group, and wherein R¹ and R² are not both hydrogen;
R³ may be an optionally substituted C₁-C₆ alkyl group, an optionally substituted C₁-C₆ alkenyl group, an optionally substituted (C₁-C₂-alkyl)-O-(C₁-C₄-alkyl) group, an optionally substituted aryl group, a phenyl or an aralkyl group with a C₁-C₄ alkyl chain;
wherein the optional substituent may be selected from the group comprising halogen, aliphatic, halo aliphatic, alicyclic, alkoxy, thioalkyl, hydroxy, cyano, aryl and nitro group;
n may be 1, 2 or 3;
which comprises the amidation of an indene compound of Formula (II) 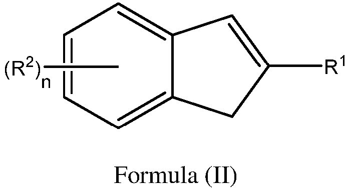 with an amidation agent selected from the compound of Formula (III), 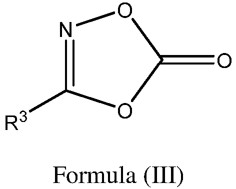
wherein the amidation may be in the presence of a hydrosilane reducing agent and a catalyst, wherein the catalyst may be a complex of a transition metal with a monodentate, bidentate or polydentate chiral ligand.

Substituents R¹ and R² may each independently be a C₁-C₄ alkyl group and n may be 1. Substituents R¹ and R² may be methyl and n may be 1.

Substituent R³ may be selected from the group consisting of methyl, ethyl, tert-butyl, tert-amyl, isopropyl, methoxymethyl, ethoxymethyl, isopropoxymethyl, methoxyethyl, ethoxyethyl, phenyl, tolyl, naphthyl, benzyl, cumyl, mesityl or xylyl. Substituent R³ may be selected from the group consisting of methyl, ethyl, tert-butyl, isopropyl, methoxymethyl, ethoxymethyl, isopropoxymethyl, phenyl, tolyl or benzyl.

Substituents R¹ and R² may each independently be a C₁-C₄ alkyl group, n may be 1, and substituent R³ may be selected from the group consisting of methyl, ethyl, tert-butyl, tert-amyl, isopropyl, methoxymethyl, ethoxymethyl, isopropoxymethyl, methoxyethyl, ethoxyethyl, phenyl, tolyl, naphthyl, benzyl, cumyl, mesityl or xylyl. Substituents R¹ and R² may be methyl, n may be 1 and substituent R³ may be selected from the group consisting of methyl, ethyl, tert-butyl, isopropyl, methoxymethyl, ethoxymethyl, isopropoxymethyl, phenyl, tolyl or benzyl.

The amidation may be carried out in a solvent. The solvent may be selected from the group consisting of tetrahydrofuran, 2-methyl tetrahydrofuran, cyclohexane, methyl cyclohexane, toluene, n-heptane, methyl tert-butyl ether, cyclopentyl methyl ether, chlorobenzene, diethyl ether, dichloromethane, 1,2-dichloroethane, 1,4-dioxane, 1,3-dioxolane, acetonitrile, dimethylacetamide, dimethylformamide, dimethoxymethane, dimethoxyethane, cumene, xylenes, cymenes, mesitylene and mixtures thereof. The solvent may be selected from the group consisting of dimethylacetamide, dimethylformamide, tetrahydrofuran, 2-methyl tetrahydrofuran, 1,4-dioxane, 1,3-dioxolane, dimethoxymethane, dimethoxyethane and mixtures thereof. The solvent may be selected from the group consisting of dimethylacetamide, tetrahydrofuran, 1,4-dioxane, 1,3-dioxolane, dimethoxyethane and mixtures thereof.

Substituents R¹ and R² may each independently be a C₁-C₄ alkyl group, n may be 1, substituent R³ may be selected from the group consisting of methyl, ethyl, tert-butyl, tert-amyl, isopropyl, methoxymethyl, ethoxymethyl, isopropoxymethyl, methoxyethyl, ethoxyethyl, phenyl, tolyl, naphthyl, benzyl, cumyl, mesityl or xylyl, and the solvent for the amidation reaction may be selected from the group consisting of dimethylacetamide, dimethylformamide, tetrahydrofuran, 2-methyl tetrahydrofuran, 1,4-dioxane, 1,3-dioxolane, dimethoxymethane, dimethoxyethane and mixtures thereof. Substituents R¹ and R² may be methyl, n may be 1, substituent R³ may be selected from the group consisting of methyl, ethyl, tert-butyl, isopropyl, methoxymethyl, ethoxymethyl, isopropoxymethyl, phenyl, tolyl or benzyl, and the solvent for the amidation reaction may be selected from the group consisting of dimethylacetamide, tetrahydrofuran, 1,4-dioxane, 1,3-dioxolane, dimethoxyethane and mixtures thereof.

The hydrosilane reducing agent may be selected from the group consisting of alkoxy hydrosilanes or hydrosiloxanes. The hydrosilane reducing agent may be selected from the group consisting of methyldimethoxysilane, trimethoxysilane or poly(methylhydrosiloxane).

The solvent for the amidation reaction may be selected from the group consisting of dimethylacetamide, dimethylformamide, tetrahydrofuran, 2-methyl tetrahydrofuran, 1,4-dioxane, 1,3-dioxolane, dimethoxymethane, dimethoxyethane and mixtures thereof, and the hydrosilane reducing agent may be selected from the group consisting of alkoxy hydrosilanes or hydrosiloxanes. The solvent for the amidation reaction may be selected from the group consisting of dimethylacetamide, tetrahydrofuran, 1,4-dioxane, 1,3-dioxolane, dimethoxyethane and mixtures thereof, and the hydrosilane reducing agent may be selected from the group consisting of methyldimethoxysilane, trimethoxysilane or poly(methylhydrosiloxane).

Substituents R¹ and R² may each independently be a C₁-C₄ alkyl group, n may be 1, substituent R³ may be selected from the group consisting of methyl, ethyl, tert-butyl, tert-amyl, isopropyl, methoxymethyl, ethoxymethyl, isopropoxymethyl, methoxyethyl, ethoxyethyl, phenyl, tolyl, naphthyl, benzyl, cumyl, mesityl or xylyl, the solvent for the amidation reaction may be selected from the group consisting of dimethylacetamide, dimethylformamide, tetrahydrofuran, 2-methyl tetrahydrofuran, 1,4-dioxane, 1,3-dioxolane, dimethoxymethane, dimethoxyethane and mixtures thereof, and the hydrosilane reducing agent may be selected from the group consisting of alkoxy hydrosilanes or hydrosiloxanes. Substituents R¹ and R² may be methyl, n may be 1, substituent R³ may be selected from the group consisting of methyl, ethyl, tert-butyl, isopropyl, methoxymethyl, ethoxymethyl, isopropoxymethyl, phenyl, tolyl or benzyl, the solvent for the amidation reaction may be selected from the group consisting of dimethylacetamide, tetrahydrofuran, 1,4-dioxane, 1,3-dioxolane, dimethoxyethane and mixtures thereof, and the hydrosilane reducing agent may be selected from the group consisting of methyldimethoxysilane, trimethoxysilane or poly(methylhydrosiloxane).

The transition metal of the catalyst complex may be a cationic transition metal. The transition metal may have an oxidation state of 0, +1, +2, +3, or +4. The transition metal of the catalyst complex may have a transition state of +2. The transition metal of the catalyst complex may be selected from the group including Cu²⁺, Ni²⁺, Pd²⁺, Pt²⁺, Fe²⁺, Ru²⁺, Cr²⁺, Mo²⁺, W²⁺, Co²⁺ or Mn²⁺. The transition metal of the catalyst complex may be Cu²⁺ or Ni²⁺. The transition metal of the catalyst complex may be Ni²⁺.

The solvent for the amidation reaction may be selected from the group consisting of dimethylacetamide, dimethylformamide, tetrahydrofuran, 2-methyl tetrahydrofuran, 1,4-dioxane, 1,3-dioxolane, dimethoxymethane, dimethoxyethane and mixtures thereof, the hydrosilane reducing agent may be selected from the group consisting of alkoxy hydrosilanes or hydrosiloxanes, and the transition metal of the catalyst complex may be Cu²⁺ or Ni²⁺. The solvent for the amidation reaction may be selected from the group consisting of dimethylacetamide, tetrahydrofuran, 1,4-dioxane, 1,3-dioxolane, dimethoxyethane and mixtures thereof, the hydrosilane reducing agent may be selected from the group consisting of methyldimethoxysilane, trimethoxysilane or poly(methylhydrosiloxane), and the transition metal of the catalyst complex may be Ni²⁺.

Substituents R¹ and R² may each independently be a C₁-C₄ alkyl group, n may be 1, substituent R³ may be selected from the group consisting of methyl, ethyl, tert-butyl, tert-amyl, isopropyl, methoxymethyl, ethoxymethyl, isopropoxymethyl, methoxyethyl, ethoxyethyl, phenyl, tolyl, naphthyl, benzyl, cumyl, mesityl or xylyl, the solvent for the amidation reaction may be selected from the group consisting of dimethylacetamide, dimethylformamide, tetrahydrofuran, 2-methyl tetrahydrofuran, 1,4-dioxane, 1,3-dioxolane, dimethoxymethane, dimethoxyethane and mixtures thereof, the hydrosilane reducing agent may be selected from the group consisting of alkoxy hydrosilanes or hydrosiloxanes, and the transition metal of the catalyst complex may be Cu²⁺ or Ni²⁺. Substituents R¹ and R² may be methyl, n may be 1, substituent R³ may be selected from the group consisting of methyl, ethyl, tert-butyl, isopropyl, methoxymethyl, ethoxymethyl, isopropoxymethyl, phenyl, tolyl or benzyl, the solvent for the amidation reaction may be selected from the group consisting of dimethylacetamide, tetrahydrofuran, 1,4-dioxane, 1,3-dioxolane, dimethoxyethane and mixtures thereof, the hydrosilane reducing agent may be selected from the group consisting of methyldimethoxysilane, trimethoxysilane or poly(methylhydrosiloxane), and the transition metal of the catalyst complex may be Cu²⁺ or Ni²⁺.

The catalyst may be a complex of a transition metal with a chiral ligand that may be prepared *in situ* or *ex situ.*

When the catalyst complex is prepared *in situ,* the Ni²⁺ may be added to the process as a compound selected from the group consisting of Nickel diiodide, Nickel (II) tetrafluoroborate hexahydrate, Nickel (II) bis(acetylacetonate), Nickel dichloride, Nickel dichloride hexahydrate, Nickel dibromide dimethoxyethane, Nickel difluoride tetrahydrate and Nickel (II) trifluoroacetate. When the catalyst complex is prepared *in situ,* the Ni²⁺ may be added to the process as a compound selected from the group consisting of Nickel diiodide, Nickel dichloride hexahydrate, and Nickel dibromide dimethoxyethane.

The chiral ligand of the catalyst complex may be a monodentate, bidentate or polydentate chiral ligand. The chiral ligand may be a bidentate chiral ligand. The chiral ligand may be a phosphorous-containing bidentate chiral ligand or a nitrogen-containing bidentate chiral ligand. The chiral ligand may be a nitrogen-containing bidentate chiral ligand.

The phosphorous-containing bidentate chiral ligand may be selected from the group consisting of (+)-1,2-bis((2*S*,5*S*)-2,5-diphenylphospholano)ethane, (+)-1,2-bis((2*S*,5*S*)-2,5-dipropylphospholano)ethane, (-)-1,2-bis[(2*R*,5*R*)-2,5-diethylphospho-lano]benzene, (-)-1,2-bis[(2*R*,5*R*)-2,5-dimethylphospholano]benzene, 1,2-bis(diphenyl-phosphino) ethane (DPPE), (+)-2,2-dimethyl-4,5-((diphenyl-phosphino)dimethyl)dioxolane, (2*S*,4*S*)-(-)-4-diphenyl-phosphino-2-(diphenylphosphino-methyl)pyrrolidine, 2,2'-bis(diphenyl-phosphino)-1,1'-binaphthyl) (BINAP), and (-)-1,2-bis[(2*R*,5*R*)-2,5-dimethyl-phospholano]benzene, derivatives and enantiomers thereof. The phosphorous-containing bidentate chiral ligand may be (+)-1,2-bis((2*S*,5*S*)-2,5-diphenylphospholano)ethane.

The nitrogen-containing bidentate chiral ligand may be selected from the group consisting of (3a*R*,3'a*R*,8a*S*,8'a*S*)-3a,3'a,8a,8'a-tetrahydro-8H,8'H-2,2'-biindeno[1,2-d]oxazole, (5*R*,5'*R*)-5,5'-diisobutyl-4,4',5,5'-tetrahydro-2,2'-bioxazole, (5*R*,5'*R*)-5,5'-diphenyl-4,4',5,5'-tetrahydro-2,2'-bioxazole, (5*R*,5'*R*)-5,5'-dibenzyl-4,4',5,5'-tetrahydro-2,2'-bioxazole, (5*R*,5'*R*)-5,5'-di-tert-butyl-4,4',5,5'-tetrahydro-2,2'-bioxazole, (5*R*,5'*R*)-5,5'-dimethyl-4,4',5,5'-tetrahydro-2,2'-bioxazole, (5*R*,5'*R*)-5,5'-diisopropyl-4,4',5,5'-tetra hydro-2,2'-bioxazole, derivatives and enantiomers thereof. The nitrogen-containing bidentate chiral ligand may be selected from the group consisting of (3a*R*,3'a*R*,8a*S*,8'a*S*)-3a,3'a,8a,8'a-tetrahydro-8H,8'H-2,2'-biindeno[1,2-d]oxazole, (5*R*,5'*R*)-5,5'-di-tert-butyl-4,4',5,5'-tetrahydro-2,2'-bioxazole, (5*R*,5'*R*)-5,5'-diphenyl-4,4',5,5'-tetrahydro-2,2'-bioxazole and (5*R*,5'*R*)-5,5'-dibenzyl-4,4',5,5'-tetrahydro-2,2'-bioxazole. The nitrogen-containing bidentate chiral ligand may be (3a*R*,3'a*R*,8a*S*,8'a*S*)-3a,3'a,8a,8'a-tetrahydro-8H,8'H-2,2'-biindeno[1,2-d]oxazole or (5*R*,5'*R*)-5,5'-diphenyl-4,4',5,5'-tetrahydro-2,2'-bioxazole.

When the catalyst complex is prepared *in situ,* the Ni²⁺ may be added to the process as a compound selected from the group consisting of Nickel diiodide, Nickel (II) tetrafluoroborate hexahydrate, Nickel (II) bis(acetylacetonate), Nickel dichloride, Nickel dichloride hexahydrate, Nickel dibromide dimethoxyethane, Nickel difluoride tetrahydrate and Nickel (II) trifluoroacetate, and the nitrogen-containing bidentate chiral ligand may be selected from the group consisting of (3a*R*,3'a*R*,8a*S*,8'a*S*)-3a,3'a,8a,8'a-tetrahydro-8H,8'H-2,2'-biindeno[1,2-d]oxazole, (5*R*,5'*R*)-5,5'-di-tert-butyl-4,4',5,5'-tetrahydro-2,2'-bioxazole, (5*R,*5'*R*)-5,5'-diphenyl-4,4',5,5'-tetrahydro-2,2'-bioxazole and (5*R*,5'*R*)-5,5'-dibenzyl-4,4',5,5'-tetrahydro-2,2'-bioxazole. When the catalyst complex is prepared *in situ,* the Ni²⁺ may be added to the process as a compound selected from the group consisting of Nickel diiodide, Nickel dichloride hexahydrate, and Nickel dibromide dimethoxyethane, and the nitrogen-containing bidentate chiral ligand may be (3a*R*,3'a*R*,8a*S*,8'a*S*)-3a,3'a,8a,8'a-tetrahydro-8H,8'H-2,2'-biindeno[1,2-d]oxazole or (5*R*,5'*R*)-5,5'-diphenyl-4,4',5,5'-tetrahydro-2,2'-bioxazole.

The solvent for the amidation reaction may be selected from the group consisting of dimethylacetamide, dimethylformamide, tetrahydrofuran, 2-methyl tetrahydrofuran, 1,4-dioxane, 1,3-dioxolane, dimethoxymethane, dimethoxyethane and mixtures thereof, the hydrosilane reducing agent may be selected from the group consisting of alkoxy hydrosilanes or hydrosiloxanes, and when the catalyst complex is prepared *in situ,* the Ni²⁺ may be added to the process as a compound selected from the group consisting of Nickel diiodide, Nickel (II) tetrafluoroborate hexahydrate, Nickel (II) bis(acetylacetonate), Nickel dichloride, Nickel dichloride hexahydrate, Nickel dibromide dimethoxyethane, Nickel difluoride tetrahydrate and Nickel (II) trifluoroacetate, and the nitrogen-containing bidentate chiral ligand may be selected from the group consisting of (3a*R*,3'a*R*,8a*S*,8'a*S*)-3a,3'a,8a,8'a-tetrahydro-8H,8'H-2,2'-biindeno[1,2-d]oxazole, (5*R*,5'*R*)-5,5'-di-tert-butyl-4,4',5,5'-tetrahydro-2,2'-bioxazole, (5*R*,5'*R*)-5,5'-diphenyl-4,4',5,5'-tetrahydro-2,2'-bioxazole and (5*R*,5'*R*)-5,5'-dibenzyl-4,4',5,5'-tetrahydro-2,2'-bioxazole. The solvent for the amidation reaction may be selected from the group consisting of dimethylacetamide, tetrahydrofuran, 1,4-dioxane, 1,3-dioxolane, dimethoxyethane and mixtures thereof, the hydrosilane reducing agent may be selected from the group consisting of methyldimethoxysilane, trimethoxysilane or poly(methylhydrosiloxane), and when the catalyst complex is prepared *in situ,* the Ni²⁺ may be added to the process as a compound selected from the group consisting of Nickel diiodide, Nickel dichloride hexahydrate, and Nickel dibromide dimethoxyethane, and the nitrogen-containing bidentate chiral ligand may be (3a*R*,3'a*R*,8a*S*,8'a*S*)-3a,3'a,8a,8'a-tetrahydro-8H,8'H-2,2'-biindeno[1,2-d]oxazole or (5*R*,5'*R*)-5,5'-diphenyl-4,4',5,5'-tetrahydro-2,2'-bioxazole.

Substituents R¹ and R² may each independently be a C₁-C₄ alkyl group, n may be 1, substituent R³ may be selected from the group consisting of methyl, ethyl, tert-butyl, tert-amyl, isopropyl, methoxymethyl, ethoxymethyl, isopropoxymethyl, methoxyethyl, ethoxyethyl, phenyl, tolyl, naphthyl, benzyl, cumyl, mesityl or xylyl, the solvent for the amidation reaction may be selected from the group consisting of dimethylacetamide, dimethylformamide, tetrahydrofuran, 2-methyl tetrahydrofuran, 1,4-dioxane, 1,3-dioxolane, dimethoxymethane, dimethoxyethane and mixtures thereof, the hydrosilane reducing agent may be selected from the group consisting of alkoxy hydrosilanes or hydrosiloxanes, and when the catalyst complex is prepared *in situ,* the Ni²⁺ may be added to the process as a compound selected from the group consisting of Nickel diiodide, Nickel (II) tetrafluoroborate hexahydrate, Nickel (II) bis(acetylacetonate), Nickel dichloride, Nickel dichloride hexahydrate, Nickel dibromide dimethoxyethane, Nickel difluoride tetrahydrate and Nickel (II) trifluoroacetate, and the nitrogen-containing bidentate chiral ligand may be selected from the group consisting of (3a*R*,3'a*R*,8a*S*,8'a*S*)-3a,3'a,8a,8'a-tetrahydro-8H,8'H-2,2'-biindeno[1,2-d]oxazole, (5*R*,5'*R*)-5,5'-di-tert-butyl-4,4',5,5'-tetrahydro-2,2'-bioxazole, (5*R*,5'*R*)-5,5'-diphenyl-4,4',5,5'-tetrahydro-2,2'-bioxazole and (5*R*,5'*R*)-5,5'-dibenzyl-4,4',5,5'-tetrahydro-2,2'-bioxazole. Substituents R¹ and R² may be methyl, n may be 1, substituent R³ may be selected from the group consisting of methyl, ethyl, tert-butyl, isopropyl, methoxymethyl, ethoxymethyl, isopropoxymethyl, phenyl, tolyl or benzyl, the solvent for the amidation reaction may be selected from the group consisting of dimethylacetamide, tetrahydrofuran, 1,4-dioxane, 1,3-dioxolane, dimethoxyethane and mixtures thereof, the hydrosilane reducing agent may be selected from the group consisting of methyldimethoxysilane, trimethoxysilane or poly(methylhydrosiloxane), and when the catalyst complex is prepared *in situ,* the Ni²⁺ may be added to the process as a compound selected from the group consisting of Nickel diiodide, Nickel dichloride hexahydrate, and Nickel dibromide dimethoxyethane, and the nitrogen-containing bidentate chiral ligand may be (3a*R*,3'a*R*,8a*S*,8'a*S*)-3a,3'a,8a,8'a-tetrahydro-8H,8'H-2,2'-biindeno[1,2-d]oxazole or (5*R*,5'*R*)-5,5'-diphenyl-4,4',5,5'-tetrahydro-2,2'-bioxazole.

The amidation agent may be a compound of Formula (III) wherein R³ has the meaning as hereinabove defined for the compound of Formula (I). The amidation agent may be selected from the group consisting of 3-methyl-1,4,2-dioxazol-5-one, 3-ethyl-1,4,2-dioxazol-5-one, 3-tert-butyl-1,4,2-dioxazol-5-one, 3-tert-amyl-1,4,2-dioxazol-5-one, 3-isopropyl-1,4,2-dioxazol-5-one, 3-phenyl-1,4,2-dioxazol-5-one, 3-tolyl-1,4,2-dioxazol-5-one, 3-benzyl-1,4,2-dioxazol-5-one, 3-mesityl-1,4,2-dioxazol-5-one, 3-cumyl-1,4,2-dioxazol-5-one, 3-methoxymethyl-1,4,2-dioxazol-5-one, 3-ethoxymethyl-1,4,2-dioxazol-5-one, 3-isopropoxymethyl-1,4,2-dioxazol-5-one or 3-methoxyethyl-1,4,2-dioxazol-5-one. The amidation agent may be selected from the group consisting of 3-methyl-1,4,2-dioxazol-5-one, 3-ethyl-1,4,2-dioxazol-5-one, 3-tert-butyl-1,4,2-dioxazol-5-one, 3-isopropyl-1,4,2-dioxazol-5-one, 3-phenyl-1,4,2-dioxazol-5-one, 3-tolyl-1,4,2-dioxazol-5-one, 3-benzyl-1,4,2-dioxazol-5-one, 3-methoxymethyl-1,4,2-dioxazol-5-one, 3-ethoxymethyl-1,4,2-dioxazol-5-one or 3-isopropoxymethyl-1,4,2-dioxazol-5-one.

The solvent for the amidation reaction may be selected from the group consisting of dimethylacetamide, dimethylformamide, tetrahydrofuran, 2-methyl tetrahydrofuran, 1,4-dioxane, 1,3-dioxolane, dimethoxymethane, dimethoxyethane and mixtures thereof, the hydrosilane reducing agent may be selected from the group consisting of alkoxy hydrosilanes or hydrosiloxanes, the amidation agent may be selected from the group consisting of 3-methyl-1,4,2-dioxazol-5-one, 3-ethyl-1,4,2-dioxazol-5-one, 3-tert-butyl-1,4,2-dioxazol-5-one, 3-tert-amyl-1,4,2-dioxazol-5-one, 3-isopropyl-1,4,2-dioxazol-5-one, 3-phenyl-1,4,2-dioxazol-5-one, 3-tolyl-1,4,2-dioxazol-5-one, 3-benzyl-1,4,2-dioxazol-5-one, 3-mesityl-1,4,2-dioxazol-5-one, 3-cumyl-1,4,2-dioxazol-5-one, 3-methoxymethyl-1,4,2-dioxazol-5-one, 3-ethoxymethyl-1,4,2-dioxazol-5-one, 3-isopropoxymethyl-1,4,2-dioxazol-5-one or 3-methoxyethyl-1,4,2-dioxazol-5-one, and when the catalyst complex is prepared *in situ,* the Ni²⁺ may be added to the process as a compound selected from the group consisting of Nickel diiodide, Nickel (II) tetrafluoroborate hexahydrate, Nickel (II) bis(acetylacetonate), Nickel dichloride, Nickel dichloride hexahydrate, Nickel dibromide dimethoxyethane, Nickel difluoride tetrahydrate and Nickel (II) trifluoroacetate, and the nitrogen-containing bidentate chiral ligand may be selected from the group consisting of (3a*R*,3'a*R*,8a*S*,8'a*S*)-3a,3'a,8a,8'a-tetrahydro-8H,8'H-2,2'-biindeno[1,2-d]oxazole, (5*R*,5'*R*)-5,5'-di-tert-butyl-4,4',5,5'-tetrahydro-2,2'-bioxazole, (5*R*,5'*R*)-5,5'-diphenyl-4,4',5,5'-tetrahydro-2,2'-bioxazole and (5*R*,5'*R*)-5,5'-dibenzyl-4,4',5,5'-tetrahydro-2,2'-bioxazole. The solvent for the amidation reaction may be selected from the group consisting of dimethylacetamide, tetrahydrofuran, 1,4-dioxane, 1,3-dioxolane, dimethoxyethane and mixtures thereof, the hydrosilane reducing agent may be selected from the group consisting of methyldimethoxysilane, trimethoxysilane or poly(methylhydrosiloxane), the amidation agent may be selected from the group consisting of 3-methyl-1,4,2-dioxazol-5-one, 3-ethyl-1,4,2-dioxazol-5-one, 3-tert-butyl-1,4,2-dioxazol-5-one, 3-isopropyl-1,4,2-dioxazol-5-one, 3-phenyl-1,4,2-dioxazol-5-one, 3-tolyl-1,4,2-dioxazol-5-one, 3-benzyl-1,4,2-dioxazol-5-one, 3-methoxymethyl-1,4,2-dioxazol-5-one, 3-ethoxymethyl-1,4,2-dioxazol-5-one or 3-isopropoxymethyl-1,4,2-dioxazol-5-one, and when the catalyst complex is prepared *in situ,* the Ni²⁺ may be added to the process as a compound selected from the group consisting of Nickel diiodide, Nickel dichloride hexahydrate, and Nickel dibromide dimethoxyethane, and the nitrogen-containing bidentate chiral ligand may be (3a*R*,3'a*R*,8a*S*,8'a*S*)-3a,3'a,8a,8'a-tetrahydro-8H,8'H-2,2'-biindeno[1,2-d]oxazole or (5*R*,5'*R*)-5,5'-diphenyl-4,4',5,5'-tetrahydro-2,2'-bioxazole.

Substituents R¹ and R² may each independently be a C₁-C₄ alkyl group, n may be 1, the solvent for the amidation reaction may be selected from the group consisting of dimethylacetamide, dimethylformamide, tetrahydrofuran, 2-methyl tetrahydrofuran, 1,4-dioxane, 1,3-dioxolane, dimethoxymethane, dimethoxyethane and mixtures thereof, the hydrosilane reducing agent may be selected from the group consisting of alkoxy hydrosilanes or hydrosiloxanes, the amidation agent may be selected from the group consisting of 3-methyl-1,4,2-dioxazol-5-one, 3-ethyl-1,4,2-dioxazol-5-one, 3-tert-butyl-1,4,2-dioxazol-5-one, 3-tert-amyl-1,4,2-dioxazol-5-one, 3-isopropyl-1,4,2-dioxazol-5-one, 3-phenyl-1,4,2-dioxazol-5-one, 3-tolyl-1,4,2-dioxazol-5-one, 3-benzyl-1,4,2-dioxazol-5-one, 3-mesityl-1,4,2-dioxazol-5-one, 3-cumyl-1,4,2-dioxazol-5-one, 3-methoxymethyl-1,4,2-dioxazol-5-one, 3-ethoxymethyl-1,4,2-dioxazol-5-one, 3-isopropoxymethyl-1,4,2-dioxazol-5-one or 3-methoxyethyl-1,4,2-dioxazol-5-one, and when the catalyst complex is prepared *in situ,* the Ni²⁺ may be added to the process as a compound selected from the group consisting of Nickel diiodide, Nickel (II) tetrafluoroborate hexahydrate, Nickel (II) bis(acetylacetonate), Nickel dichloride, Nickel dichloride hexahydrate, Nickel dibromide dimethoxyethane, Nickel difluoride tetrahydrate and Nickel (II) trifluoroacetate, and the nitrogen-containing bidentate chiral ligand may be selected from the group consisting of (3a*R*,3'a*R*,8a*S*,8'a*S*)-3a,3'a,8a,8'a-tetrahydro-8H,8'H-2,2'-biindeno[1,2-d]oxazole, (5*R*,5'*R*)-5,5'-di-tert-butyl-4,4',5,5'-tetrahydro-2,2'-bioxazole, (5*R*,5'*R*)-5,5'-diphenyl-4,4',5,5'-tetrahydro-2,2'-bioxazole and (5*R*,5'*R*)-5,5'-dibenzyl-4,4',5,5'-tetrahydro-2,2'-bioxazole. Substituents R¹ and R² may be methyl, n may be 1, the solvent for the amidation reaction may be selected from the group consisting of dimethylacetamide, tetrahydrofuran, 1,4-dioxane, 1,3-dioxolane, dimethoxyethane and mixtures thereof, the hydrosilane reducing agent may be selected from the group consisting of methyldimethoxysilane, trimethoxysilane or poly(methylhydrosiloxane), the amidation agent may be selected from the group consisting of 3-methyl-1,4,2-dioxazol-5-one, 3-ethyl-1,4,2-dioxazol-5-one, 3-tert-butyl-1,4,2-dioxazol-5-one, 3-isopropyl-1,4,2-dioxazol-5-one, 3-phenyl-1,4,2-dioxazol-5-one, 3-tolyl-1,4,2-dioxazol-5-one, 3-benzyl-1,4,2-dioxazol-5-one, 3-methoxymethyl-1,4,2-dioxazol-5-one, 3-ethoxymethyl-1,4,2-dioxazol-5-one or 3-isopropoxymethyl-1,4,2-dioxazol-5-one, and when the catalyst complex is prepared *in situ,* the Ni²⁺ may be added to the process as a compound selected from the group consisting of Nickel diiodide, Nickel dichloride hexahydrate, and Nickel dibromide dimethoxyethane, and the nitrogen-containing bidentate chiral ligand may be (3*aR*,3'*aR*,8*aS*,8'*aS*)-3a,3'a,8a,8'a-tetrahydro-8H,8'H-2,2'-biindeno[1,2-d]oxazole or (5*R*,5'*R*)-5,5'-diphenyl-4,4',5,5'-tetrahydro-2,2'-bioxazole.

The process may include the addition of an additional protic solvent to the solvent of the reaction. The additional protic solvent may be water or an alcohol or a glycol. The additional protic solvent may be water or an alcohol selected from the group consisting of methanol, ethanol, isopropanol, isobutanol or tert-butanol. The additional protic solvent may be tert-butanol.

The additional protic solvent may be water or an alcohol or a glycol, the solvent for the amidation reaction may be selected from the group consisting of dimethylacetamide, dimethylformamide, tetrahydrofuran, 2-methyl tetrahydrofuran, 1,4-dioxane, 1,3-dioxolane, dimethoxymethane, dimethoxyethane and mixtures thereof. The additional protic solvent may be water or an alcohol selected from the group consisting of methanol, ethanol, isopropanol, isobutanol or tert-butanol, and the solvent for the amidation reaction may be selected from the group consisting of dimethylacetamide, tetrahydrofuran, 1,4-dioxane, 1,3-dioxolane, dimethoxyethane and mixtures thereof.

The additional protic solvent may be water or an alcohol or a glycol, the solvent for the amidation reaction may be selected from the group consisting of dimethylacetamide, dimethylformamide, tetrahydrofuran, 2-methyl tetrahydrofuran, 1,4-dioxane, 1,3-dioxolane, dimethoxymethane, dimethoxyethane and mixtures thereof, the hydrosilane reducing agent may be selected from the group consisting of alkoxy hydrosilanes or hydrosiloxanes, the amidation agent may be selected from the group consisting of 3-methyl-1,4,2-dioxazol-5-one, 3-ethyl-1,4,2-dioxazol-5-one, 3-tert-butyl-1,4,2-dioxazol-5-one, 3-tert-amyl-1,4,2-dioxazol-5-one, 3-isopropyl-1,4,2-dioxazol-5-one, 3-phenyl-1,4,2-dioxazol-5-one, 3-tolyl-1,4,2-dioxazol-5-one, 3-benzyl-1,4,2-dioxazol-5-one, 3-mesityl-1,4,2-dioxazol-5-one, 3-cumyl-1,4,2-dioxazol-5-one, 3-methoxymethyl-1,4,2-dioxazol-5-one, 3-ethoxymethyl-1,4,2-dioxazol-5-one, 3-isopropoxymethyl-1,4,2-dioxazol-5-one or 3-methoxyethyl-1,4,2-dioxazol-5-one, and when the catalyst complex is prepared *in situ,* the Ni²⁺ may be added to the process as a compound selected from the group consisting of Nickel diiodide, Nickel (II) tetrafluoroborate hexahydrate, Nickel (II) bis(acetylacetonate), Nickel dichloride, Nickel dichloride hexahydrate, Nickel dibromide dimethoxyethane, Nickel difluoride tetrahydrate and Nickel (II) trifluoroacetate, and the nitrogen-containing bidentate chiral ligand may be selected from the group consisting of (3a*R*,3'a*R*,8a*S*,8'a*S*)-3a,3'a,8a,8'a-tetrahydro-8H,8'H-2,2'-biindeno[1,2-d]oxazole, (5*R*,5'*R*)-5,5'-di-tert-butyl-4,4',5,5'-tetrahydro-2,2'-bioxazole, (5*R*,5*'R*)-5,5'-diphenyl-4,4',5,5'-tetrahydro-2,2'-bioxazole and (5*R*,5'*R*)-5,5'-dibenzyl-4,4',5,5'-tetrahydro-2,2'-bioxazole. The additional protic solvent may be water or an alcohol selected from the group consisting of methanol, ethanol, isopropanol, isobutanol or tert-butanol, the solvent for the amidation reaction may be selected from the group consisting of dimethylacetamide, tetrahydrofuran, 1,4-dioxane, 1,3-dioxolane, dimethoxyethane and mixtures thereof, the hydrosilane reducing agent may be selected from the group consisting of methyldimethoxysilane, trimethoxysilane or poly(methylhydrosiloxane), the amidation agent may be selected from the group consisting of 3-methyl-1,4,2-dioxazol-5-one, 3-ethyl-1,4,2-dioxazol-5-one, 3-tert-butyl-1,4,2-dioxazol-5-one, 3-isopropyl-1,4,2-dioxazol-5-one, 3-phenyl-1,4,2-dioxazol-5-one, 3-tolyl-1,4,2-dioxazol-5-one, 3-benzyl-1,4,2-dioxazol-5-one, 3-methoxymethyl-1,4,2-dioxazol-5-one, 3-ethoxymethyl-1,4,2-dioxazol-5-one or 3-isopropoxymethyl-1,4,2-dioxazol-5-one, and when the catalyst complex is prepared *in situ,* the Ni²⁺ may be added to the process as a compound selected from the group consisting of Nickel diiodide, Nickel dichloride hexahydrate, and Nickel dibromide dimethoxyethane, and the nitrogen-containing bidentate chiral ligand may be (3*aR*,3'*aR*,8*aS*,8'*aS*)-3a,3'a,8a,8'a-tetrahydro-8H,8'H-2,2'-biindeno[1,2-d]oxazole or (5*R*,5'*R*)-5,5'-diphenyl-4,4',5,5'-tetrahydro-2,2'-bioxazole.

Substituents R¹ and R² may each independently be a C₁-C₄ alkyl group, n may be 1, the additional protic solvent may be water or an alcohol or a glycol, the solvent for the amidation reaction may be selected from the group consisting of dimethylacetamide, dimethylformamide, tetrahydrofuran, 2-methyl tetrahydrofuran, 1,4-dioxane, 1,3-dioxolane, dimethoxymethane, dimethoxyethane and mixtures thereof, the hydrosilane reducing agent may be selected from the group consisting of alkoxy hydrosilanes or hydrosiloxanes, the amidation agent may be selected from the group consisting of 3-methyl-1,4,2-dioxazol-5-one, 3-ethyl-1,4,2-dioxazol-5-one, 3-tert-butyl-1,4,2-dioxazol-5-one, 3-tert-amyl-1,4,2-dioxazol-5-one, 3-isopropyl-1,4,2-dioxazol-5-one, 3-phenyl-1,4,2-dioxazol-5-one, 3-tolyl-1,4,2-dioxazol-5-one, 3-benzyl-1,4,2-dioxazol-5-one, 3-mesityl-1,4,2-dioxazol-5-one, 3-cumyl-1,4,2-dioxazol-5-one, 3-methoxymethyl-1,4,2-dioxazol-5-one, 3-ethoxymethyl-1,4,2-dioxazol-5-one, 3-isopropoxymethyl-1,4,2-dioxazol-5-one or 3-methoxyethyl-1,4,2-dioxazol-5-one, and when the catalyst complex is prepared *in situ,* the Ni²⁺ may be added to the process as a compound selected from the group consisting of Nickel diiodide, Nickel (II) tetrafluoroborate hexahydrate, Nickel (II) bis(acetylacetonate), Nickel dichloride, Nickel dichloride hexahydrate, Nickel dibromide dimethoxyethane, Nickel difluoride tetrahydrate and Nickel (II) trifluoroacetate, and the nitrogen-containing bidentate chiral ligand may be selected from the group consisting of (3a*R*,3'a*R*,8a*S*,8'a*S*)-3a,3'a,8a,8'a-tetrahydro-8H,8'H-2,2'-biindeno[1,2-d]oxazole, (5*R*,5'*R*)-5,5'-di-tert-butyl-4,4',5,5'-tetrahydro-2,2'-bioxazole, (5*R*,5*'R*)-5,5'-diphenyl-4,4',5,5'-tetrahydro-2,2'-bioxazole and (5*R*,5'*R*)-5,5'-dibenzyl-4,4',5,5'-tetrahydro-2,2'-bioxazole. Substituents R¹ and R² may be methyl, n may be 1, the additional protic solvent may be water or an alcohol selected from the group consisting of methanol, ethanol, isopropanol, isobutanol or tert-butanol, the solvent for the amidation reaction may be selected from the group consisting of dimethylacetamide, tetrahydrofuran, 1,4-dioxane, 1,3-dioxolane, dimethoxyethane and mixtures thereof, the hydrosilane reducing agent may be selected from the group consisting of methyldimethoxysilane, trimethoxysilane or poly(methylhydrosiloxane), the amidation agent may be selected from the group consisting of 3-methyl-1,4,2-dioxazol-5-one, 3-ethyl-1,4,2-dioxazol-5-one, 3-tert-butyl-1,4,2-dioxazol-5-one, 3-isopropyl-1,4,2-dioxazol-5-one, 3-phenyl-1,4,2-dioxazol-5-one, 3-tolyl-1,4,2-dioxazol-5-one, 3-benzyl-1,4,2-dioxazol-5-one, 3-methoxymethyl-1,4,2-dioxazol-5-one, 3-ethoxymethyl-1,4,2-dioxazol-5-one or 3-isopropoxymethyl-1,4,2-dioxazol-5-one, and when the catalyst complex is prepared *in situ,* the Ni²⁺ may be added to the process as a compound selected from the group consisting of Nickel diiodide, Nickel dichloride hexahydrate, and Nickel dibromide dimethoxyethane, and the nitrogen-containing bidentate chiral ligand may be (3a*R*,3'a*R*,8a*S*,8'a*S*)-3a,3'a,8a,8'a-tetrahydro-8H,8'H-2,2'-biindeno[1,2-d]oxazole or (5*R*,5'*R*)-5,5'-diphenyl-4,4',5,5'-tetrahydro-2,2'-bioxazole.

The process may include the addition of an additive selected from the group including sodium iodide (NaI), potassium iodide (KI), lithium iodide (LiI) and cesium iodide (CsI).

The amidation reaction may take place at a temperature in the range between about 0°C to 100°C, about 25°C to 75°C, about 45°C to 70°C.

The amidation reaction may take place under atmospheric pressure. The amidation reaction may take place under a nitrogen atmosphere. The amidation reaction may take place under a nitrogen atmosphere at atmospheric pressure.

The amidation reaction may take place under atmospheric pressure at a temperature in the range between about 0°C to 100°C, about 25°C to 75°C, about 45°C to 70°C. The amidation reaction may take place under a nitrogen atmosphere at a temperature in the range between about 0°C to 100°C, about 25°C to 75°C, about 45°C to 70°C. The amidation reaction may take place under a nitrogen atmosphere at atmospheric pressure and at a temperature in the range between about 0°C to 100°C, about 25°C to 75°C, about 45°C to 70°C

The compound of Formula (I) may be a substituted (1*R*,2*S*)-1-amido-2,6-dimethylindane of Formula (IV), 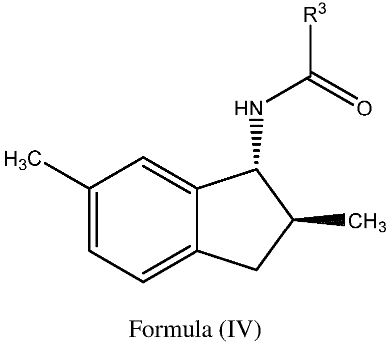 wherein the R³ substituent has the meaning as hereinabove defined for the compound of Formula (I), i.e. R³ may be an optionally substituted C₁-C₆ alkyl group, an optionally substituted C₁-C₆ alkenyl group, an optionally substituted (C₁-C₂-alkyl)-O-(C₁-C₄-alkyl) group, an optionally substituted aryl group, a phenyl or an aralkyl group with a C₁-C₄ alkyl chain;
wherein the optional substituent may be selected from the group comprising halogen, aliphatic, halo aliphatic, alicyclic, alkoxy, thioalkyl, hydroxy, cyano, aryl and nitro group.

The substituent R³ in compound of Formula (IV) may be selected from the group consisting of methyl, ethyl, tert-butyl, tert-amyl, isopropyl, methoxymethyl, ethoxymethyl, isopropoxymethyl, methoxyethyl, ethoxyethyl, phenyl, tolyl, naphthyl, benzyl, cumyl, mesityl or xylyl. The substituent R³ in compound of Formula (IV) may be selected from the group consisting of methyl, ethyl, tert-butyl, isopropyl, methoxymethyl, ethoxymethyl, isopropoxymethyl, phenyl, tolyl or benzyl.

The substituent R³ in compound of Formula (IV) may be selected from the group consisting of methyl, ethyl, tert-butyl, tert-amyl, isopropyl, methoxymethyl, ethoxymethyl, isopropoxymethyl, methoxyethyl, ethoxyethyl, phenyl, tolyl, naphthyl, benzyl, cumyl, mesityl or xylyl, and the solvent for the amidation reaction may be selected from the group consisting of dimethylacetamide, dimethylformamide, tetrahydrofuran, 2-methyl tetrahydrofuran, 1,4-dioxane, 1,3-dioxolane, dimethoxymethane, dimethoxyethane and mixtures thereof. The substituent R³ in compound of Formula (IV) may be selected from the group consisting of methyl, ethyl, tert-butyl, isopropyl, methoxymethyl, ethoxymethyl, isopropoxymethyl, phenyl, tolyl or benzyl, and the solvent for the amidation reaction may be selected from the group consisting of dimethylacetamide, tetrahydrofuran, 1,4-dioxane, 1,3-dioxolane, dimethoxyethane and mixtures thereof.

The substituent R³ in compound of Formula (IV) may be selected from the group consisting of methyl, ethyl, tert-butyl, tert-amyl, isopropyl, methoxymethyl, ethoxymethyl, isopropoxymethyl, methoxyethyl, ethoxyethyl, phenyl, tolyl, naphthyl, benzyl, cumyl, mesityl or xylyl, the solvent for the amidation reaction may be selected from the group consisting of dimethylacetamide, dimethylformamide, tetrahydrofuran, 2-methyl tetrahydrofuran, 1,4-dioxane, 1,3-dioxolane, dimethoxymethane, dimethoxyethane and mixtures thereof, and the hydrosilane reducing agent may be selected from the group consisting of alkoxy hydrosilanes or hydrosiloxanes. The substituent R³ in compound of Formula (IV) may be selected from the group consisting of methyl, ethyl, tert-butyl, isopropyl, methoxymethyl, ethoxymethyl, isopropoxymethyl, phenyl, tolyl or benzyl, the solvent for the amidation reaction may be selected from the group consisting of dimethylacetamide, tetrahydrofuran, 1,4-dioxane, 1,3-dioxolane, dimethoxyethane and mixtures thereof, and the hydrosilane reducing agent may be selected from the group consisting of methyldimethoxysilane, trimethoxysilane or poly(methylhydrosiloxane).

The substituent R³ in compound of Formula (IV) may be selected from the group consisting of methyl, ethyl, tert-butyl, tert-amyl, isopropyl, methoxymethyl, ethoxymethyl, isopropoxymethyl, methoxyethyl, ethoxyethyl, phenyl, tolyl, naphthyl, benzyl, cumyl, mesityl or xylyl, the solvent for the amidation reaction may be selected from the group consisting of dimethylacetamide, dimethylformamide, tetrahydrofuran, 2-methyl tetrahydrofuran, 1,4-dioxane, 1,3-dioxolane, dimethoxymethane, dimethoxyethane and mixtures thereof, the hydrosilane reducing agent may be selected from the group consisting of alkoxy hydrosilanes or hydrosiloxanes, and the transition metal of the catalyst complex may be Cu²⁺ or Ni²⁺. The substituent R³ in compound of Formula (IV) may be selected from the group consisting of methyl, ethyl, tert-butyl, isopropyl, methoxymethyl, ethoxymethyl, isopropoxymethyl, phenyl, tolyl or benzyl, the solvent for the amidation reaction may be selected from the group consisting of dimethylacetamide, tetrahydrofuran, 1,4-dioxane, 1,3-dioxolane, dimethoxyethane and mixtures thereof, the hydrosilane reducing agent may be selected from the group consisting of methyldimethoxysilane, trimethoxysilane or poly(methylhydrosiloxane), and the transition metal of the catalyst complex may be Cu²⁺ or Ni²⁺.

The substituent R³ in compound of Formula (IV) may be selected from the group consisting of methyl, ethyl, tert-butyl, tert-amyl, isopropyl, methoxymethyl, ethoxymethyl, isopropoxymethyl, methoxyethyl, ethoxyethyl, phenyl, tolyl, naphthyl, benzyl, cumyl, mesityl or xylyl, the solvent for the amidation reaction may be selected from the group consisting of dimethylacetamide, dimethylformamide, tetrahydrofuran, 2-methyl tetrahydrofuran, 1,4-dioxane, 1,3-dioxolane, dimethoxymethane, dimethoxyethane and mixtures thereof, the hydrosilane reducing agent may be selected from the group consisting of alkoxy hydrosilanes or hydrosiloxanes, and when the catalyst complex is prepared *in situ,* the Ni²⁺ may be added to the process as a compound selected from the group consisting of Nickel diiodide, Nickel (II) tetrafluoroborate hexahydrate, Nickel (II) bis(acetylacetonate), Nickel dichloride, Nickel dichloride hexahydrate, Nickel dibromide dimethoxyethane, Nickel difluoride tetrahydrate and Nickel (II) trifluoroacetate, and the nitrogen-containing bidentate chiral ligand may be selected from the group consisting of (3a*R*,3'a*R*,8a*S*,8'a*S*)-3a,3'a,8a,8'a-tetrahydro-8H,8'H-2,2'-biindeno[1,2-d]oxazole, (5*R*,5'*R*)-5,5'-di-tert-butyl-4,4',5,5'-tetrahydro-2,2'-bioxazole, (5*R*,5'*R*)-5,5'-diphenyl-4,4',5,5'-tetrahydro-2,2'-bioxazole and (5*R*,5'*R*)-5,5'-dibenzyl-4,4',5,5'-tetrahydro-2,2'-bioxazole. The substituent R³ in compound of Formula (IV) may be selected from the group consisting of methyl, ethyl, tert-butyl, isopropyl, methoxymethyl, ethoxymethyl, isopropoxymethyl, phenyl, tolyl or benzyl, the solvent for the amidation reaction may be selected from the group consisting of dimethylacetamide, tetrahydrofuran, 1,4-dioxane, 1,3-dioxolane, dimethoxyethane and mixtures thereof, the hydrosilane reducing agent may be selected from the group consisting of methyldimethoxysilane, trimethoxysilane or poly(methylhydrosiloxane), and when the catalyst complex is prepared *in situ,* the Ni²⁺ may be added to the process as a compound selected from the group consisting of Nickel diiodide, Nickel dichloride hexahydrate, and Nickel dibromide dimethoxyethane, and the nitrogen-containing bidentate chiral ligand may be (3a*R*,3'a*R*,8a*S*,8'a*S*)-3a,3'a,8a,8'a-tetrahydro-8H,8'H-2,2'-biindeno[1,2-d]oxazole or (5*R*,5'*R*)-5,5'-diphenyl-4,4',5,5'-tetrahydro-2,2'-bioxazole.

The compound of Formula (I) may be a substituted (1*R*,2*S*)-1-amido-2,6-dimethylindane of Formula (IV), the solvent for the amidation reaction may be selected from the group consisting of dimethylacetamide, dimethylformamide, tetrahydrofuran, 2-methyl tetrahydrofuran, 1,4-dioxane, 1,3-dioxolane, dimethoxymethane, dimethoxyethane and mixtures thereof, the hydrosilane reducing agent may be selected from the group consisting of alkoxy hydrosilanes or hydrosiloxanes, the amidation agent may be selected from the group consisting of 3-methyl-1,4,2-dioxazol-5-one, 3-ethyl-1,4,2-dioxazol-5-one, 3-tert-butyl-1,4,2-dioxazol-5-one, 3-tert-amyl-1,4,2-dioxazol-5-one, 3-isopropyl-1,4,2-dioxazol-5-one, 3-phenyl-1,4,2-dioxazol-5-one, 3-tolyl-1,4,2-dioxazol-5-one, 3-benzyl-1,4,2-dioxazol-5-one, 3-mesityl-1,4,2-dioxazol-5-one, 3-cumyl-1,4,2-dioxazol-5-one, 3-methoxymethyl-1,4,2-dioxazol-5-one, 3-ethoxymethyl-1,4,2-dioxazol-5-one, 3-isopropoxymethyl-1,4,2-dioxazol-5-one or 3-methoxyethyl-1,4,2-dioxazol-5-one, and when the catalyst complex is prepared *in situ,* the Ni²⁺ may be added to the process as a compound selected from the group consisting of Nickel diiodide, Nickel (II) tetrafluoroborate hexahydrate, Nickel (II) bis(acetylacetonate), Nickel dichloride, Nickel dichloride hexahydrate, Nickel dibromide dimethoxyethane, Nickel difluoride tetrahydrate and Nickel (II) trifluoroacetate, and the nitrogen-containing bidentate chiral ligand may be selected from the group consisting of (3a*R*,3'a*R*,8a*S*,8'a*S*)-3a,3'a,8a,8'a-tetrahydro-8H,8'H-2,2'-biindeno[1,2-d]oxazole, (5*R*,5'*R*)-5,5'-di-tert-butyl-4,4',5,5'-tetrahydro-2,2'-bioxazole, (5*R*,5'*R*)-5,5'-diphenyl-4,4',5,5'-tetrahydro-2,2'-bioxazole and (5*R*,5'*R*)-5,5'-dibenzyl-4,4',5,5'-tetrahydro-2,2'-bioxazole. The compound of Formula (I) may be a substituted (1*R*,2*S*)-1-amido-2,6-dimethylindane of Formula (IV), the solvent for the amidation reaction may be selected from the group consisting of dimethylacetamide, tetrahydrofuran, 1,4-dioxane, 1,3-dioxolane, dimethoxyethane and mixtures thereof, the hydrosilane reducing agent may be selected from the group consisting of methyldimethoxysilane, trimethoxysilane or poly(methylhydrosiloxane), the amidation agent may be selected from the group consisting of 3-methyl-1,4,2-dioxazol-5-one, 3-ethyl-1,4,2-dioxazol-5-one, 3-tert-butyl-1,4,2-dioxazol-5-one, 3-isopropyl-1,4,2-dioxazol-5-one, 3-phenyl-1,4,2-dioxazol-5-one, 3-tolyl-1,4,2-dioxazol-5-one, 3-benzyl-1,4,2-dioxazol-5-one, 3-methoxymethyl-1,4,2-dioxazol-5-one, 3-ethoxymethyl-1,4,2-dioxazol-5-one or 3-isopropoxymethyl-1,4,2-dioxazol-5-one, and when the catalyst complex is prepared *in situ,* the Ni²⁺ may be added to the process as a compound selected from the group consisting of Nickel diiodide, Nickel dichloride hexahydrate, and Nickel dibromide dimethoxyethane, and the nitrogen-containing bidentate chiral ligand may be (3*aR*,3'*aR*,8*aS*,8'*aS*)-3a,3'a,8a,8'a-tetrahydro-8H,8'H-2,2'-biindeno[1,2-d]oxazole or (5*R*,5'*R*)-5,5'-diphenyl-4,4',5,5'-tetrahydro-2,2'-bioxazole.

The compound of Formula (I) may be a substituted (1*R*,2*S*)-1-amido-2,6-dimethylindane of Formula (IV), the additional protic solvent may be water or an alcohol or a glycol, the solvent for the amidation reaction may be selected from the group consisting of dimethylacetamide, dimethylformamide, tetrahydrofuran, 2-methyl tetrahydrofuran, 1,4-dioxane, 1,3-dioxolane, dimethoxymethane, dimethoxyethane and mixtures thereof, the hydrosilane reducing agent may be selected from the group consisting of alkoxy hydrosilanes or hydrosiloxanes, the amidation agent may be selected from the group consisting of 3-methyl-1,4,2-dioxazol-5-one, 3-ethyl-1,4,2-dioxazol-5-one, 3-tert-butyl-1,4,2-dioxazol-5-one, 3-tert-amyl-1,4,2-dioxazol-5-one, 3-isopropyl-1,4,2-dioxazol-5-one, 3-phenyl-1,4,2-dioxazol-5-one, 3-tolyl-1,4,2-dioxazol-5-one, 3-benzyl-1,4,2-dioxazol-5-one, 3-mesityl-1,4,2-dioxazol-5-one, 3-cumyl-1,4,2-dioxazol-5-one, 3-methoxymethyl-1,4,2-dioxazol-5-one, 3-ethoxymethyl-1,4,2-dioxazol-5-one, 3-isopropoxymethyl-1,4,2-dioxazol-5-one or 3-methoxyethyl-1,4,2-dioxazol-5-one, and when the catalyst complex is prepared *in situ,* the Ni²⁺ may be added to the process as a compound selected from the group consisting of Nickel diiodide, Nickel (II) tetrafluoroborate hexahydrate, Nickel (II) bis(acetylacetonate), Nickel dichloride, Nickel dichloride hexahydrate, Nickel dibromide dimethoxyethane, Nickel difluoride tetrahydrate and Nickel (II) trifluoroacetate, and the nitrogen-containing bidentate chiral ligand may be selected from the group consisting of (3a*R*,3'a*R*,8a*S*,8'a*S*)-3a,3'a,8a,8'a-tetrahydro-8H,8'H-2,2'-biindeno[1,2-d]oxazole, (5*R*,5'*R*)-5,5'-di-tert-butyl-4,4',5,5'-tetrahydro-2,2'-bioxazole, (5*R*,5'*R*)-5,5'-diphenyl-4,4',5,5'-tetrahydro-2,2'-bioxazole and (5*R*,5'*R*)-5,5'-dibenzyl-4,4',5,5'-tetrahydro-2,2'-bioxazole. The compound of Formula (I) may be a substituted (1*R*,2*S*)-1-amido-2,6-dimethylindane of Formula (IV), the additional protic solvent may be water or an alcohol selected from the group consisting of methanol, ethanol, isopropanol, isobutanol or tert-butanol, the solvent for the amidation reaction may be selected from the group consisting of dimethylacetamide, tetrahydrofuran, 1,4-dioxane, 1,3-dioxolane, dimethoxyethane and mixtures thereof, the hydrosilane reducing agent may be selected from the group consisting of methyldimethoxysilane, trimethoxysilane or poly(methylhydrosiloxane), the amidation agent may be selected from the group consisting of 3-methyl-1,4,2-dioxazol-5-one, 3-ethyl-1,4,2-dioxazol-5-one, 3-tert-butyl-1,4,2-dioxazol-5-one, 3-isopropyl-1,4,2-dioxazol-5-one, 3-phenyl-1,4,2-dioxazol-5-one, 3-tolyl-1,4,2-dioxazol-5-one, 3-benzyl-1,4,2-dioxazol-5-one, 3-methoxymethyl-1,4,2-dioxazol-5-one, 3-ethoxymethyl-1,4,2-dioxazol-5-one or 3-isopropoxymethyl-1,4,2-dioxazol-5-one, and when the catalyst complex is prepared *in situ,* the Ni²⁺ may be added to the process as a compound selected from the group consisting of Nickel diiodide, Nickel dichloride hexahydrate, and Nickel dibromide dimethoxyethane, and the nitrogen-containing bidentate chiral ligand may be (3*aR*,3'*aR*,8*aS*,8'*aS*)-3a,3'a,8a,8'a-tetrahydro-8H,8'H-2,2'-biindeno[1,2-d]oxazole or (5*R*,5'*R*)-5,5'-diphenyl-4,4',5,5'-tetrahydro-2,2'-bioxazole.

### Compounds of Formula (IV)

A second aspect of the invention relates to a compound of Formula (IV), 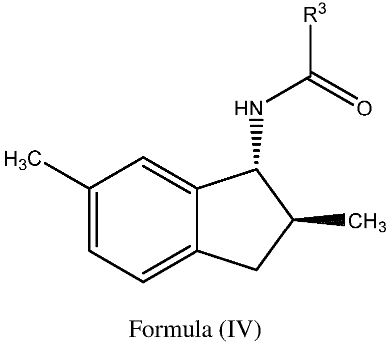 wherein R³ may be an optionally substituted C₁-C₆ alkyl group, an optionally substituted C₁-C₆ alkenyl group, an optionally substituted (C₁-C₂-alkyl)-O-(C₁-C₄-alkyl) group, an aryl group selected from the group consisting of tolyl, naphthyl or mesityl, or an aralkyl group with a C₁-C₄ alkyl chain;
wherein the optional substituent may be selected from the group comprising halogen, aliphatic, halo aliphatic, alicyclic, alkoxy, thioalkyl, hydroxy, cyano, aryl and nitro group.

The substituent R³ in compound of Formula (IV) may be selected from the group consisting of methyl, ethyl, tert-butyl, tert-amyl, isopropyl, methoxymethyl, ethoxymethyl, isopropoxymethyl, methoxyethyl, ethoxyethyl, tolyl, naphthyl, mesityl, benzyl, cumyl or xylyl. The substituent R³ in compound of Formula (IV) may be selected from the group consisting of methyl, ethyl, tert-butyl, isopropyl, methoxymethyl, ethoxymethyl, isopropoxymethyl, tolyl or benzyl.

### Process for the preparation of compounds of Formula (II)

A third aspect of the invention relates to a process for the preparation of a compound of Formula (II). The compound of Formula (II) may be prepared by a process that comprises the dehydration of an indanol compound of Formula (V) 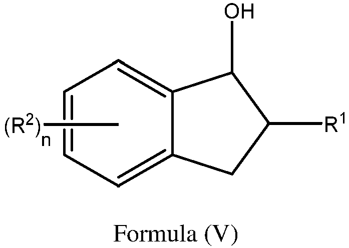
wherein R¹ and R² substituents have the meaning as hereinabove defined, i.e. R¹ and R² may each independently be hydrogen or an optionally substituted C₁-C₄ alkyl group, and wherein R¹ and R² are not both hydrogen;
wherein the optional substituent may be selected from the group comprising halogen, aliphatic, halo aliphatic, alicyclic, alkoxy, thioalkyl, hydroxy, cyano, aryl and nitro group;
n may be 1, 2 or 3.

The compound of Formula (V) may be 2,6-dimethylindan-1-ol of Formula (VI)

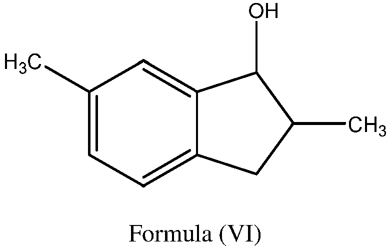

The dehydration may be carried out in a solvent. The dehydration solvent may be selected from the group consisting of toluene, chlorobenzene, dichloroethane and xylene. The dehydration solvent may be toluene.

The compound of Formula (V) may be 2,6-dimethylindan-1-ol of Formula (VI), and the dehydration solvent may be selected from the group consisting of toluene, chlorobenzene, dichloroethane and xylene. The compound of Formula (V) may be 2,6-dimethylindan-1-ol of Formula (VI), and the dehydration solvent may be toluene.

The dehydration may be carried out in the presence of a catalyst. The catalyst may be selected from the group consisting of p-toluene sulfonic acid, pyridinium p-toluene sulfonic acid, sulfosalicylic acid, ethane sulphonic acid, methanesulfonic acid, benzene sulfonic acid, camphorsulfonic acid, p-brominesulphonic acid, sulfuric acid, trifluoromethanesulfonic acid and hydrochloric acid.

The dehydration solvent may be selected from the group consisting of toluene, chlorobenzene, dichloroethane and xylene and the catalyst for the dehydration reaction may be selected from the group consisting of p-toluene sulfonic acid, pyridinium p-toluene sulfonic acid, sulfosalicylic acid, ethane sulphonic acid, methanesulfonic acid, benzene sulfonic acid, camphorsulfonic acid, p-brominesulphonic acid, sulfuric acid, trifluoromethanesulfonic acid and hydrochloric acid.

The compound of Formula (V) may be 2,6-dimethylindan-1-ol of Formula (VI), the dehydration solvent may be selected from the group consisting of toluene, chlorobenzene, dichloroethane and xylene, and the catalyst for the dehydration reaction may be selected from the group consisting of p-toluene sulfonic acid, pyridinium p-toluene sulfonic acid, sulfosalicylic acid, ethane sulphonic acid, methanesulfonic acid, benzene sulfonic acid, camphorsulfonic acid, p-brominesulphonic acid, sulfuric acid, trifluoromethanesulfonic acid and hydrochloric acid. The compound of Formula (V) may be 2,6-dimethylindan-1-ol of Formula (VI), the dehydration solvent may be toluene, and the catalyst for the dehydration reaction may be selected from the group consisting of p-toluene sulfonic acid, pyridinium p-toluene sulfonic acid, sulfosalicylic acid, ethane sulphonic acid, methanesulfonic acid, benzene sulfonic acid, camphorsulfonic acid, p-brominesulphonic acid, sulfuric acid, trifluoromethanesulfonic acid and hydrochloric acid.

### Process for the preparation of (1R,2S)-1-amino-2,6-dimethylindane

The compound of Formula (VII) is (1*R*,2*S*)-1-amino-2,6-dimethylindane, an important intermediate used in the preparation of Indaziflam, as described in WO 2009/077059 and US 2004/0157739 incorporated herein by reference in its entirety.

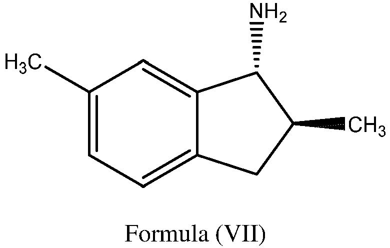

A fourth aspect of the invention is the preparation of the compound of Formula (VII) by hydrolysis of a compound of Formula (IV) in water with a hydrolyzing agent which is a base or an acid, 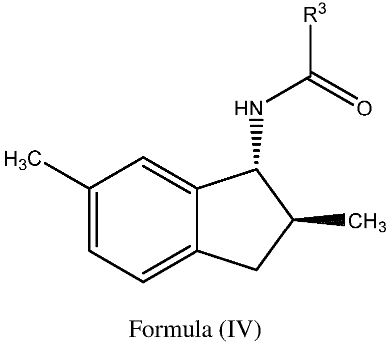 wherein the substituent R³ in compound of Formula (IV) may be an optionally substituted C₁-C₆ alkyl group, an optionally substituted C₁-C₆ alkenyl group, an optionally substituted (C₁-C₂-alkyl)-O-(C₁-C₄-alkyl) group, an optionally substituted aryl group, a phenyl or an aralkyl group with a C₁-C₄ alkyl chain;
wherein the optional substituent may be selected from the group comprising halogen, aliphatic, halo aliphatic, alicyclic, alkoxy, thioalkyl, hydroxy, cyano, aryl and nitro group.

The hydrolyzing agent may be a base. The hydrolyzing agent may be a base selected from the group consisting of an alkali metal, alkaline earth metal or ammonium hydroxide, hydride, alkoxide, carbonate, bicarbonate, phosphate, hydrogen- or dihydrogenphosphate, or an amine or mixtures thereof. The hydrolyzing agent may be a base selected from the group consisting of an alkali metal or alkaline earth metal hydroxide, alkoxide, carbonate, bicarbonate, an amine or mixtures thereof. The hydrolyzing agent may be a base selected from the group consisting of sodium hydroxide, potassium hydroxide, lithium hydroxide, triethanolamine, ammonia, hydrazine, dimethylamine, diethylamine or isopropylamine.

### Process for the preparation of Indaziflam

A fifth aspect of the invention is a process for the preparation of Indaziflam, the process comprising preparing a compound of Formula (IV) according to a hereinabove disclosed process.

A sixth aspect of the invention is a process for preparation of Indaziflam comprising:
a) preparing compound of Formula (IV) according to a hereinabove disclosed process;
b) providing reaction conditions for preparation of Indaziflam.

The reaction conditions in step (b) include, but are not limited, to hydrolysis for the preparation of the compound of Formula (VII), nucleophilic addition reaction and cyclization to obtain Indaziflam.

A seventh aspect of the invention is a process for the preparation of Indaziflam, the process comprising:
a. reacting a compound of Formula (IV) or the compound of Formula (VII) prepared according to a hereinabove disclosed process, with 1-cyanoguanidine in the presence of at least one solvent to obtain (1*R*,2*S*)-1-(bisguanidino)-2,6-dimethylindane; and
b. reacting said (1*R*,2*S*)-1-(bisguanidino)-2,6-dimethylindane with methyl-(2*R*)-2-fluoropropanoate in the presence of a base and in the presence of a solvent,
wherein when the reacting compound is a compound of Formula (IV), the compound obtained from the reaction of the compound of Formula (IV) with 1-cyanoguanidine is hydrolyzed with water and a hydrolyzing agent to obtain (1*R*,2*S*)-1-(bisguanidino)-2,6-dimethylindane before reacting with methyl-(2*R*)-2-fluoropropanoate.

The hydrolyzing agent may be a hydrolyzing agent as hereinabove disclosed for the process of preparation of the compound of Formula (VII).

A process for the preparation of Indaziflam, the process comprising:
a. reacting the compound of Formula (VII) prepared according to a hereinabove disclosed process, with 1-cyanoguanidine in the presence of at least one solvent to obtain (1*R*,2*S*)-1-(bisguanidino)-2,6-dimethylindane; and
b. reacting said (1*R*,2*S*)-1-(bisguanidino)-2,6-dimethylindane with methyl-(2*R*)-2-fluoropropanoate in the presence of a base and in the presence of a solvent.

An eighth aspect of the invention is a process for the preparation of Indaziflam, the process comprising:
a. reacting a compound of Formula (IV) or the compound of Formula (VII) prepared according to a hereinabove disclosed process, with 1-cyanoguanidine and a tri(alkyl) aluminum (III) salt in the presence of a protic solvent selected from the group consisting of alcohols or amines, to obtain a biguanidino-aluminum complex; and
b. reacting said biguanidino-aluminum complex with methyl-(2*R*)-2-fluoropropanoate, wherein when the reacting compound is a compound of Formula (IV), the biguanidino-aluminum complex obtained from the reaction of the compound of Formula (IV) with 1-cyanoguanidine and the tri(alkyl) aluminum (III) salt is hydrolyzed with water and a hydrolyzing agent before reacting with methyl-(2R)-2-fluoropropanoate.

The hydrolyzing agent may be a hydrolyzing agent as hereinabove disclosed for the process of preparation of the compound of Formula (VII).

A process for the preparation of Indaziflam, the process comprising:
a. reacting the compound of Formula (VII) prepared according to a hereinabove disclosed process, with 1-cyanoguanidine and a tri(alkyl) aluminum (III) salt in the presence of a protic solvent selected from the group consisting of alcohols or amines, to obtain a biguanidino-aluminum complex; and
b. reacting said biguanidino-aluminum complex with methyl-(2*R*)-2-fluoropropanoate.

The following examples are included for illustrative purposes only and should not be construed as limitations on the invention claimed herein.

### EXAMPLES

### Example 1: Synthesis of compounds of Formula (IV)

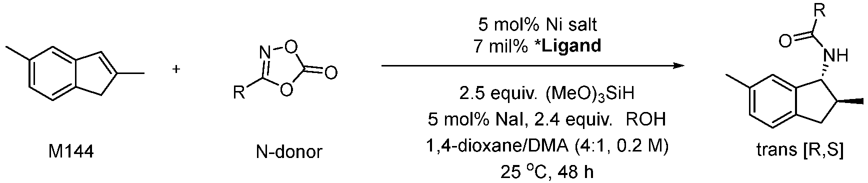

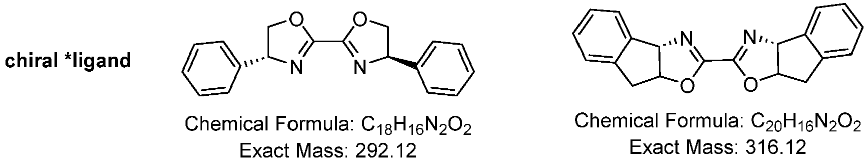

### Example 2: Ligand screening

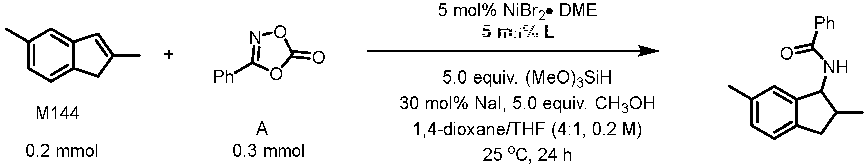

| Ligand | e.e. | Conversion |
|---|---|---|
| 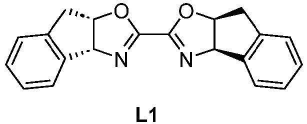 | 95% | 62% |
| 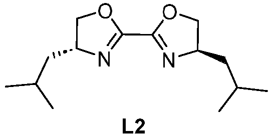 | 79% | 70% |
| 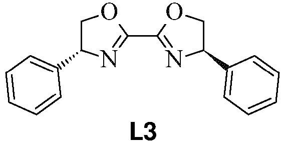 | 91% | 66% |
| 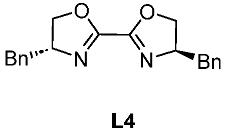 | 76% | 61% |

| | | |
|---|---|---|
| e.e: enantiomeric excess | | |

### Example 3: Solvent screening

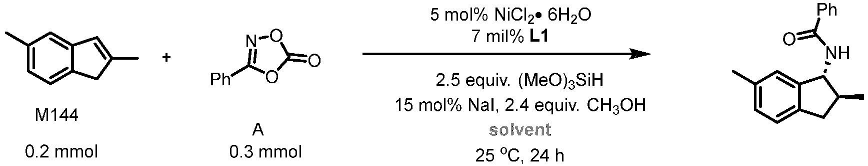

| **solvents** | **e.e. (%)** |
|---|---|
| tetrahydrofuran | 97 |
| dimethylacetamide | 98 |
| 1,4-dioxane | 99 |
| Dimethoxyethane (DME) | 98 |
| 1,3-Dioxolane | 97 |

| | |
|---|---|
| e.e: enantiomeric excess | |

### Example 4: Nickel catalyst screening

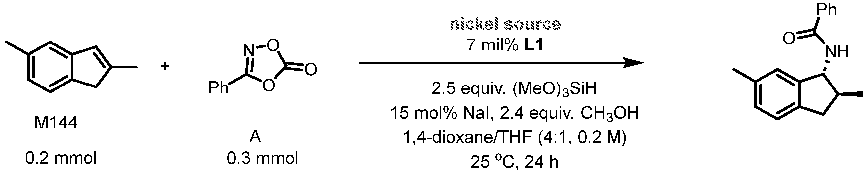

| **Nickel source** | **e.e. (%)** |
|---|---|
| NiCl₂ ·6H₂O | 97 |
| NiI₂ | 98 |
| NiBr₂ ·DME | 98 |

| | |
|---|---|
| e.e: enantiomeric excess | |

### Example 5: Protic solvent screening

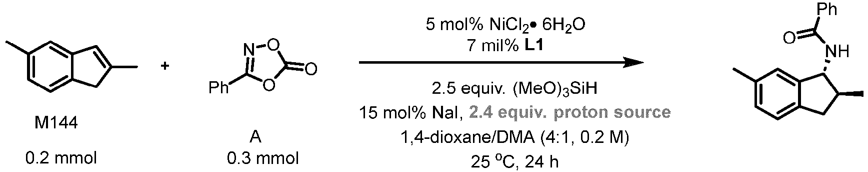

| **Protic solvent or proton source** | **Conversion (%)** |
|---|---|
| MeOH | 73 |
| EtOH | 75 |
| isobutanol | 70 |
| isopropanol | 78 |
| tert-butanol | 72 |

## Claims

1. A process for the preparation of an amide compound of Formula (I): 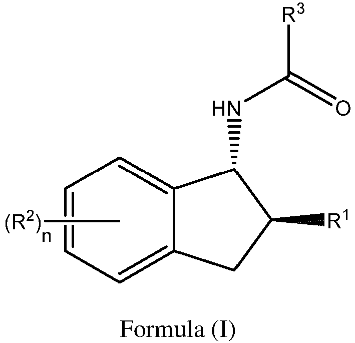 wherein:
R¹ and R² are each independently hydrogen or an optionally substituted C₁-C₄ alkyl group, and wherein R¹ and R² are not both hydrogen;
R³ is an optionally substituted C₁-C₆ alkyl group, an optionally substituted C₁-C₆ alkenyl group, an optionally substituted (C₁-C₂-alkyl)-O-(C₁-C₄-alkyl) group, an optionally substituted aryl group, a phenyl or an aralkyl group with a C₁-C₄ alkyl chain;
wherein the optional substituent is selected from the group comprising halogen, aliphatic, halo aliphatic, alicyclic, alkoxy, thioalkyl, hydroxy, cyano, aryl and nitro group;
n is 1, 2 or 3;
which comprises the amidation of an indene compound of Formula (II) 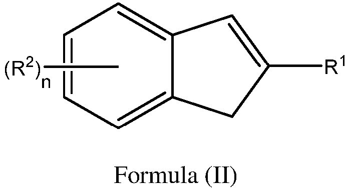 with an amidation agent selected from the compound of Formula (III), 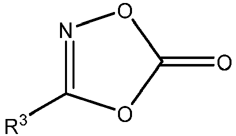 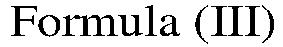 wherein the amidation is in the presence of a hydrosilane reducing agent and a catalyst,
wherein the catalyst is a complex of a transition metal with a monodentate, bidentate or polydentate chiral ligand.

2. The process according to claim 1, wherein R¹ and R² are each independently C₁-C₄ alkyl group and n is 1.

3. The process according to any of the previous claims, wherein R³ is selected from the group consisting of methyl, ethyl, tert-butyl, tert-amyl, isopropyl, methoxymethyl, ethoxymethyl, isopropoxymethyl, methoxyethyl, ethoxyethyl, phenyl, tolyl, naphthyl, benzyl, cumyl, mesityl or xylyl.

4. The process according to any of previous claims, wherein the amidation agent is selected from the group consisting of 3-methyl-1,4,2-dioxazol-5-one, 3-ethyl-1,4,2-dioxazol-5-one, 3-tert-butyl-1,4,2-dioxazol-5-one, 3-tert-amyl-1,4,2-dioxazol-5-one, 3-isopropyl-1,4,2-dioxazol-5-one, 3-phenyl-1,4,2-dioxazol-5-one, 3-tolyl-1,4,2-dioxazol-5-one, 3-benzyl-1,4,2-dioxazol-5-one, 3-mesityl-1,4,2-dioxazol-5-one, 3-cumyl-1,4,2-dioxazol-5-one, 3-methoxymethyl-1,4,2-dioxazol-5-one, 3-ethoxymethyl-1,4,2-dioxazol-5-one, 3-isopropoxymethyl-1,4,2-dioxazol-5-one or 3-methoxyethyl-1,4,2-dioxazol-5-one.

5. The process according to any of previous claims, wherein the amide compound of Formula (I) is a substituted (1R,2S)-1-amido-2,6-dimethylindane of Formula (IV),

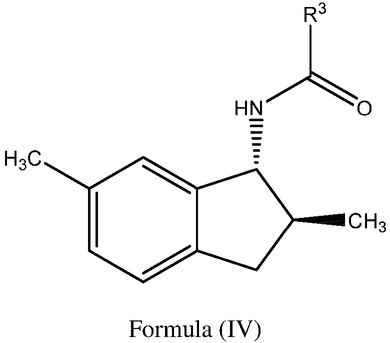

6. The process according to claim 5, wherein R³ is selected from the group consisting of methyl, ethyl, tert-butyl, tert-amyl, isopropyl, methoxymethyl, ethoxymethyl, isopropoxymethyl, methoxyethyl, ethoxyethyl, phenyl, tolyl, naphthyl, benzyl, cumyl, mesityl or xylyl.

7. A compound of Formula (IV), 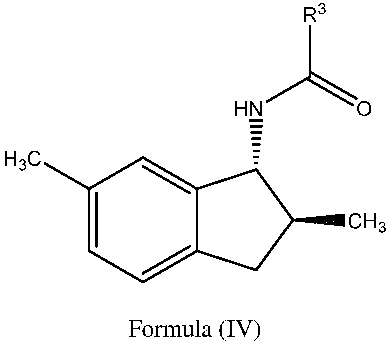
wherein R³ is an optionally substituted C₁-C₆ alkyl group, an optionally substituted C₁-C₆ alkenyl group, an optionally substituted (C₁-C₂-alkyl)-O-(C₁-C₄-alkyl) group, an aryl group selected from the group consisting of tolyl, naphthyl or mesityl, or an aralkyl group with a C₁-C₄ alkyl chain;
wherein the optional substituent is selected from the group comprising halogen, aliphatic, halo aliphatic, alicyclic, alkoxy, thioalkyl, hydroxy, cyano, aryl and nitro group.

8. The compound of Formula (IV) according to claim 7, wherein R³ is selected from the group consisting of methyl, ethyl, tert-butyl, tert-amyl, isopropyl, methoxymethyl, ethoxymethyl, isopropoxymethyl, methoxyethyl, ethoxyethyl, tolyl, naphthyl, mesityl, benzyl, cumyl or xylyl.

9. A process for the preparation of an amide compound of Formula (I): 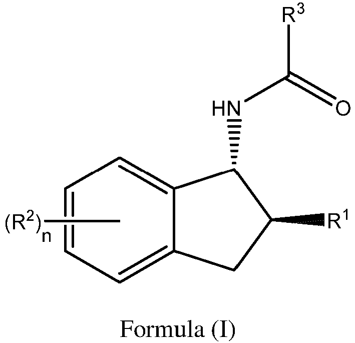 which comprises:
b) a first step of dehydration of an indanol compound of Formula (V) 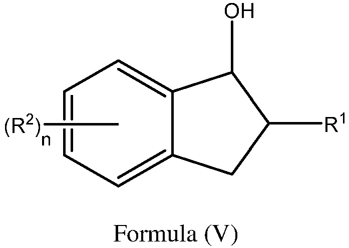 to prepare a compound of Formula (II) 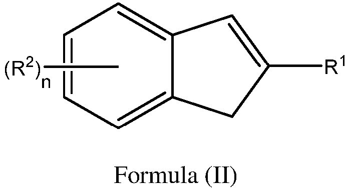 b) a second step of amidation of the indene compound of Formula (II) according to any one of claims 1 to 6;
wherein:
R¹ and R² are each independently hydrogen or an optionally substituted C₁-C₄ alkyl group, and wherein R¹ and R² are not both hydrogen;
R³ is an optionally substituted C₁-C₆ alkyl group, an optionally substituted C₁-C₆ alkenyl group, an optionally substituted (C₁-C₂-alkyl)-O-(C₁-C₄-alkyl) group, an optionally substituted aryl group, a phenyl or an aralkyl group with a C₁-C₄ alkyl chain;
wherein the optional substituent is selected from the group comprising halogen, aliphatic, halo aliphatic, alicyclic, alkoxy, thioalkyl, hydroxy, cyano, aryl and nitro group;
n is 1, 2 or 3.

10. The process according to claim 9, wherein the amide compound of Formula (I) is a substituted (1*R*,2*S*)-1-amido-2,6-dimethylindane of Formula (IV), 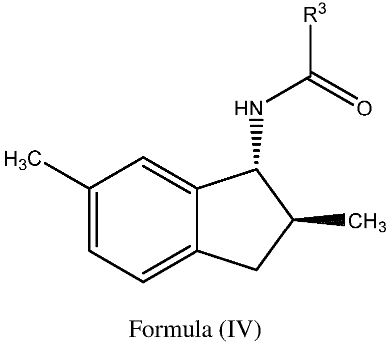 and the indanol compound of Formula (V) is 2,6-dimethylindan-1-ol of Formula (VI)

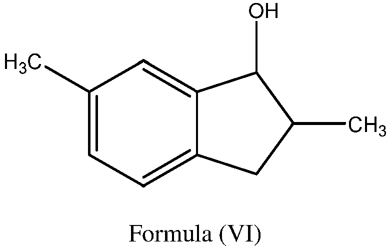

11. A process for the preparation of the compound of Formula (VII), (1R,2S)-1-amino-2,6-dimethylindane, 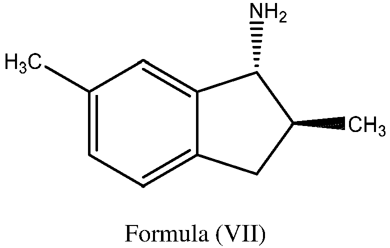 by hydrolysis of a compound of Formula (IV) in water with a hydrolyzing agent which is a base or an acid, 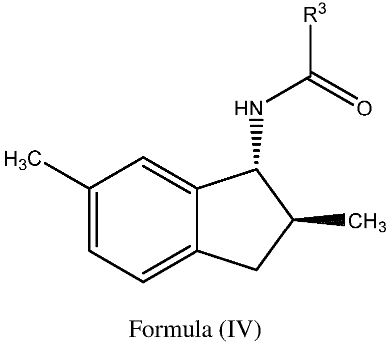 wherein the compound of Formula (IV) is prepared according to any one of claims 1 to 6;
wherein the substituent R³ in compound of Formula (IV) is an optionally substituted C₁-C₆ alkyl group, an optionally substituted C₁-C₆ alkenyl group, an optionally substituted (C₁-C₂-alkyl)-O-(C₁-C₄-alkyl) group, an optionally substituted aryl group, a phenyl or an aralkyl group with a C₁-C₄ alkyl chain;
wherein the optional substituent is selected from the group comprising halogen, aliphatic, halo aliphatic, alicyclic, alkoxy, thioalkyl, hydroxy, cyano, aryl and nitro group.

12. The process according to claim 11, wherein R³ is selected from the group consisting of methyl, ethyl, tert-butyl, tert-amyl, isopropyl, methoxymethyl, ethoxymethyl, isopropoxymethyl, methoxyethyl, ethoxyethyl, phenyl, tolyl, naphthyl, benzyl, cumyl, mesityl or xylyl.

13. A process for the preparation of Indaziflam, wherein the process comprises the preparation of a compound of Formula (IV) according to any one of claims 1 to 6.

14. A process for the preparation of Indaziflam, the process comprising:
a. reacting a compound of Formula (IV) prepared according to any one of claims 1 to 6 or the compound of Formula (VII) prepared according to any one of claims 11 to 12, with 1-cyanoguanidine in the presence of at least one solvent to obtain (1*R*,2*S*)-1-(bisguanidino)-2,6-dimethylindane; and
b. reacting said (1*R*,2*S*)-1-(bisguanidino)-2,6-dimethylindane with methyl-(2*R*)-2-fluoropropanoate in the presence of a base and in the presence of a solvent,
wherein when the reacting compound is a compound of Formula (IV), the compound obtained from the reaction of the compound of Formula (IV) with 1-cyanoguanidine is hydrolyzed with water and a hydrolyzing agent to obtain (1*R*,2*S*)-1-(bisguanidino)-2,6-dimethylindane before reacting with methyl-(2*R*)-2-fluoropropanoate.

15. A process for the preparation of Indaziflam, the process comprising:
a. reacting a compound of Formula (IV) prepared according to any one of claims 1 to 6 or the compound of Formula (VII) prepared according to any one of claims 11 to 12, with 1-cyanoguanidine and a tri(alkyl) aluminum (III) salt in the presence of a protic solvent selected from the group consisting of alcohols or amines, to obtain a biguanidino-aluminum complex; and
b. reacting said biguanidino-aluminum complex with methyl-(2R)-2-fluoropropanoate, wherein when the reacting compound is a compound of Formula (IV), the biguanidino-aluminum complex obtained from the reaction of the compound of Formula (IV) with 1-cyanoguanidine and the tri(alkyl) aluminum (III) salt is hydrolyzed with water and a hydrolyzing agent before reacting with methyl-(2*R*)-2-fluoropropanoate.
